Europäisches Patentamt

European Patent Office

Office européen des brevets

(19)

(11) **EP 0 997 536 A2**

(12) **EUROPEAN PATENT APPLICATION**

(43) Date of publication:
03.05.2000 Bulletin 2000/18

(51) Int. Cl.⁷: **C12Q 1/68**, C12N 9/90

(21) Application number: **99118075.3**

(22) Date of filing: **24.09.1999**

(84) Designated Contracting States:
**AT BE CH CY DE DK ES FI FR GB GR IE IT LI LU
MC NL PT SE**
Designated Extension States:
**AL LT LV MK RO SI**

(30) Priority: **24.09.1998 JP 27033798**

(71) Applicant:
**MARINE BIOTECHNOLOGY INSTITUTE CO.,
LTD.
Bunkyo-ku, Tokyo 113-0033 (JP)**

(72) Inventors:
• **Yamamoto, Satoshi,
Kamaishi Laboratory
Kamaishi-shi, Iwate 026-0001 (JP)**

• **Atsumi, Mika ,
Kamaishi Laboratory
Kamaishi-shi, Iwate 026-0001 (JP)**
• **Kasai, Hiroaki,
Kamaishi Laboratory
Kamaishi-shi, Iwate 026-0001 (JP)**
• **Hamada, Thoru,
Kamaishi Laboratory
Kamaishi-shi, Iwate 026-0001 (JP)**
• **Nakamura, Shoko,
Kamaishi Laboratory
Kamaishi-shi, Iwate 026-0001 (JP)**

(74) Representative:
**VOSSIUS & PARTNER
Siebertstrasse 4
81675 München (DE)**

(54) **Method of identifying and detecting eukaryotes using type II topoisomerase gene as indicator**

(57)    A eukaryote is identified and detected based on the base sequence of an intron-containing region in type II topoisomerase gene. Also, a eukaryote is identified and detected by amplifying a type II topoisomerase gene fragment of the eukaryote using specific primers or probes and identifying the eukaryote based on the sequence of the amplified DNA. An organism can therefore be identified or detected rapidly and precisely.

EP 0 997 536 A2

Printed by Xerox (UK) Business Services
2.16.7 (HRS)/3.6

**Description**

FIELD OF THE INVENTION

This invention relates to a method of identifying and detecting a eukaryote using a type II topoisomerase gene as an indicator. The invention is beneficial in various industrial fields involving identification and detection of eukaryotic organisms and authentication and monitoring based on the results obtained thereby, such as medicine, epidemiology, food industry, agriculture, forestry, fisheries, and environmental engineering.

BACKGROUND ART

Eukaryotes are roughly divided into Animalia, Plantae, Protista, and Fungi. Eukaryotes have been identified based on their morphological characteristics as main criteria. Morphological identification is difficult to automate and heavily relies on an expert who knows well-versed in the minute structural characteristics of the eukaryotes whose identification is sought. Further, since morphology is significantly influenced by the surroundings, morphology is not regarded as a definitive indicator. On the other hand, the difficulty of identification has taken a large role in allowing the spread of counterfeits (i.e., complete fakes sold under the name of a specific species, or a mixture of a specific species and a fake species sold as a pure species). Under these circumstances, introduction of a DNA authentication technique has been under study as a desirable way to ensure that a certificate may be awarded to only those species which have been authenticated by authorities. Such authentication technique has been attempted for, e.g., rice, etc. belonging to Plantae. Similarly, with respect to organisms belonging to Animalia, it is expected that environmental monitoring based on the number of larvae of invertebrates living in seawater, such as shrimps, crabs, and shellfishes, could be carried out more easily by utilizing the DNA sequence. Further, identification based on DNA base sequences will be effective in monitoring algae belonging to Protista which cause a red tide or a water bloom, or detecting yeasts pathogenic to humans, which belong to Fungi.

Among DNA sequences useful for these purposes, it is considered that those reflecting the phylogenetic relation among organisms will provide fewer errors in identification and detection of organisms or classification, authentication and monitoring based on the results of the identification. From this viewpoint, it has already been attempted to develop an identification technique based on an SSU rRNA (small subunit ribosomal RNA) base sequence. An rRNA gene is one of genes suited for development of organism identification, because its sequence is very easy to obtain. However, rRNA is a biopolymer showing very slow evolution, having maintained its basic structure over nearly three billion years following the first occurrence of life. Therefore, there is almost no appreciable difference in the rRNA base sequence among similar or closely-related species, which tends to make rRNA unsuitable for phylogenetic analysis of closely-related organisms. Molecular phylogenetic analysis of structural genes encoding proteins which show more rapid evolution than SSU rRNA is expected to elucidate the phylogeny of more closely-related organisms. A method for identifying a bacterial species belonging to Procaryota by means of the base sequence of the DNA gyrase (type II topoisomerase) gene has been developed. Also, a method of identifying eukaryotes using type II topoisomerases has been mentioned in U.S. Patent 5,645,994, but a sufficient base sequence for permitting precise identification and detection of eukaryotes cannot be obtained by the method disclosed in this patent, as is evident from the fact that the sequence actually obtained according to their method is of procaryote origin, with no sequence of eukaryote origin found. Besides, in identifying a bacterial species, there is another type II topoisomerase in addition to the bacteria type II topoisomerase, DNA gyrase. The present inventors have recognized that these deficiency result in a serious error in identifying a new strain. Accordingly, from these considerations at least, the technique of U.S. Patent 5,645,994 alone is not sufficient for adequate identification and detection of eukaryotes.

An object of invention was to identify and detect a eukaryote or classify, authenticate or monitor the eukaryote according to the results of identification and detection based on the base sequence of the type II topoisomerase gene of the eukaryote. However, the number of available base sequences of type II topoisomerase genes that has been published is too small to successfully apply the present method. Accordingly, another object of the present invention is to develop a method for easily and rapidly determine the base sequence of type II topoisomerase genes of various eukaryotic organisms.

SUMMARY OF THE INVENTION

The present invention is based on the following findings.

1) Determination of the base sequence of an intron-containing region of a type II topoisomerase enables detection of extremely minute differences among individuals (for example, differences in strain or line or growth group among individuals of the same species).

2) Use of primers that are not disclosed in U.S. Patent No. 5,645,994 enables identification and detection of eukaryotes with unexpectedly higher accuracy.

Based in part on the foregoing finding, the objects of the present invention have been attained in a first embodiment providing a method of identifying or detecting a eukaryote based on the base sequence of an intron-containing region in a type II topoisomerase gene.

The object of the present invention are also provided in a second embodiment of a method of identifying or detecting a eukaryote which comprises amplifying the DNA of the eukaryote by a polymerase chain reaction (hereinafter referred to as "PCR") or cDNA cloning using, as at least one of primers or as a probe, an oligonucleotide which comprises a base sequence coding for at least a part of (or the whole of) the following amino acid sequences (a) to (k) and functions as a substantial primer or probe, and identifying the eukaryote based on the base sequence of the amplified DNA:

    (a) Asp-Asp-Glu-Lys-Lys-Val-Thr-Gly
    (b) Lys-Lys-Lys-Asn-Lys-Gly-Gly
    (c) Met-Ile-Met-Thr-Asp-Gln-Asp-Gln-Asp-Gly
    (d) Glu-Glu-Phe-Ile-Thr-Pro-Ile-Val
    (e) Pro-Glu-Phe-Glu-Glu-Trp-Lys
    (f) Arg-Pro-Asp-(Thr or Ser)-Tyr-Ile-Gly
    (g) Leu-(Tyr or Leu or Phe)-Lys-Ile-Phe-Asp-Glu-Ile
    (h) Asn-Gly-(Tyr or Phe)-Gly-Ala-Lys-Leu
    (i) Thr-Ile-Lys-Gly-Gly-Thr-His-Val
    (j) Asp-Ala-Asn-Lys-Ala-Gly-The-Lys
    (k) Val-Asn-Gly-Ala-Glu-Gly-Ile

BRIEF DESCRIPTION OF THE DRAWINGS

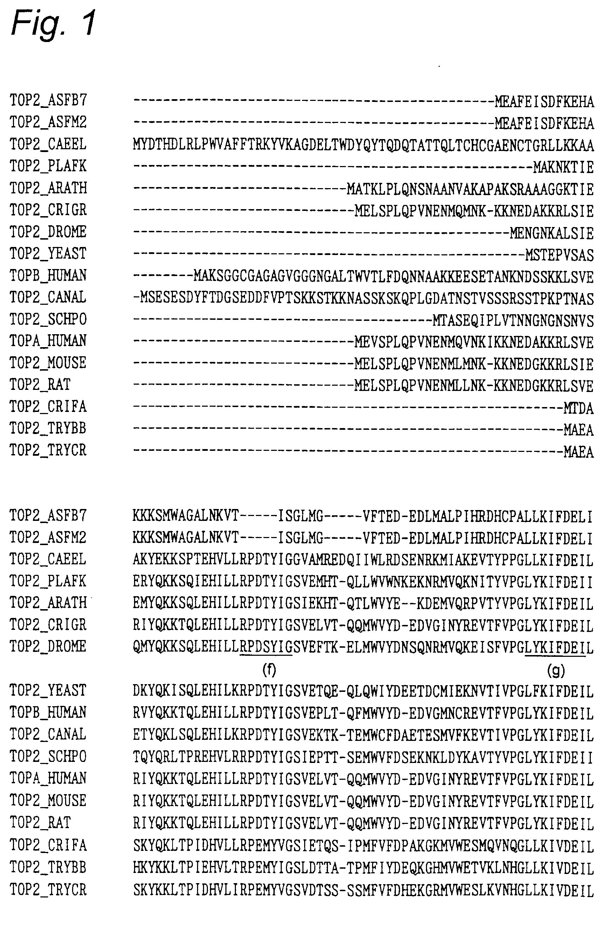

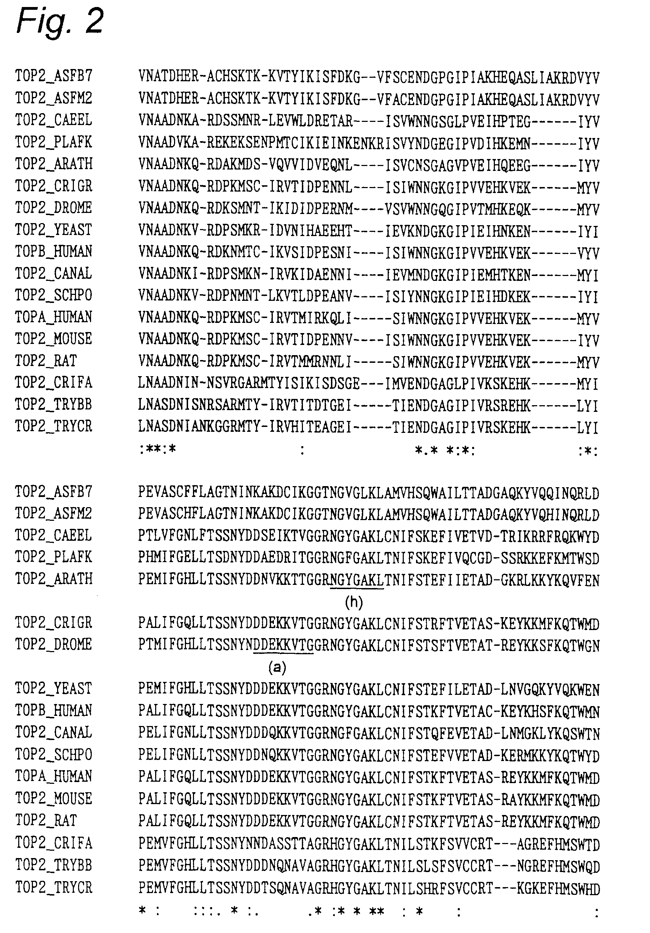

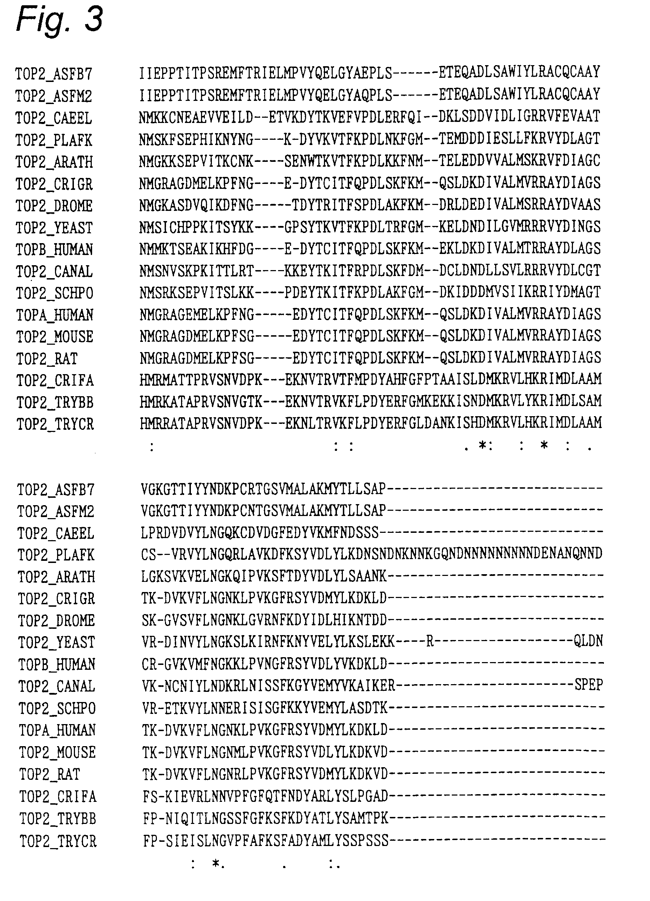

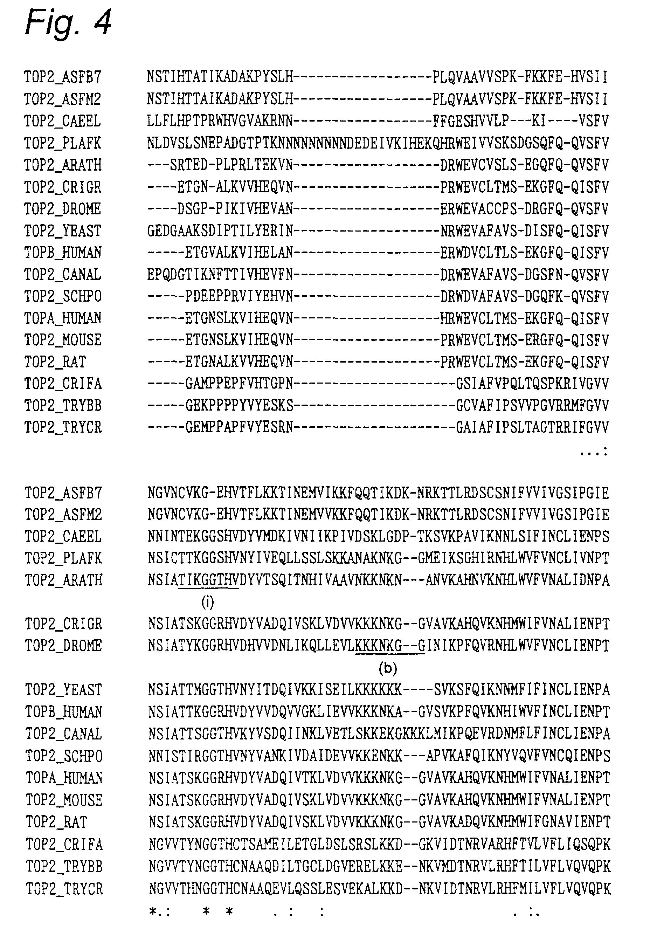

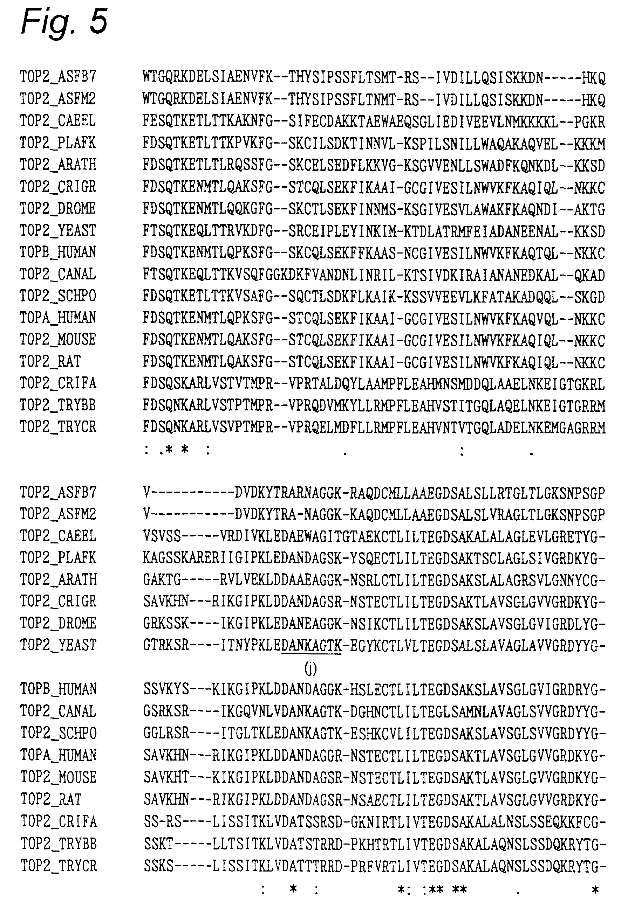

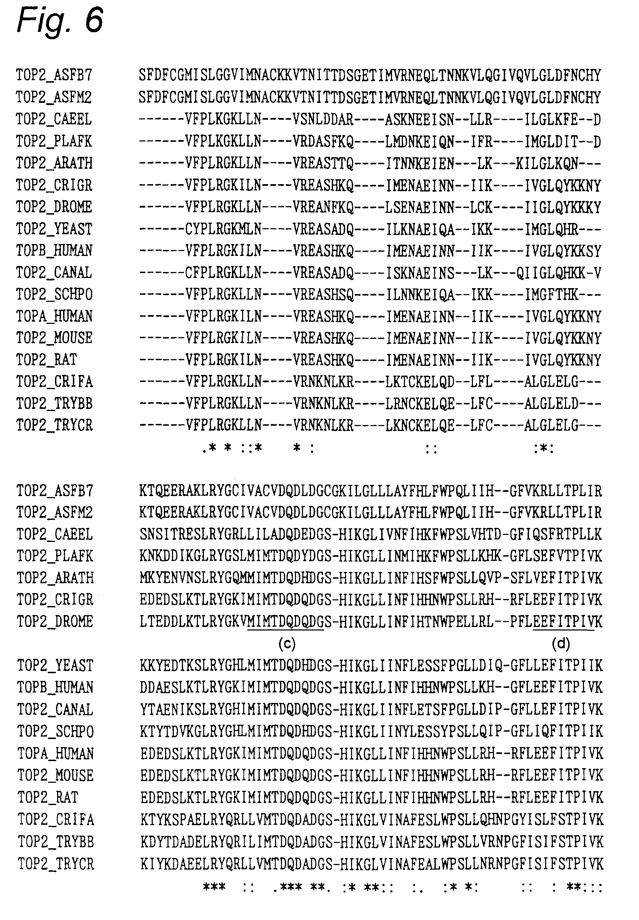

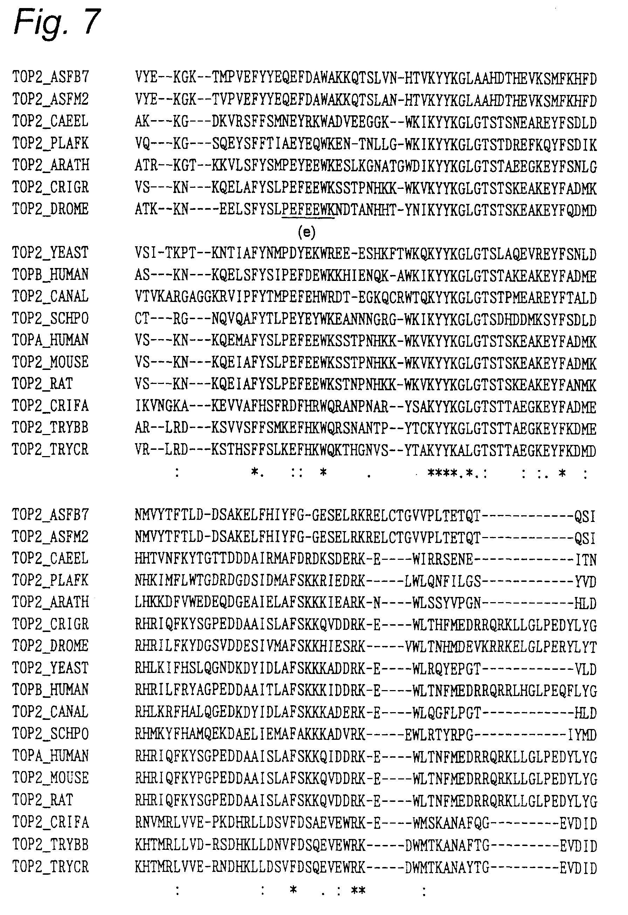

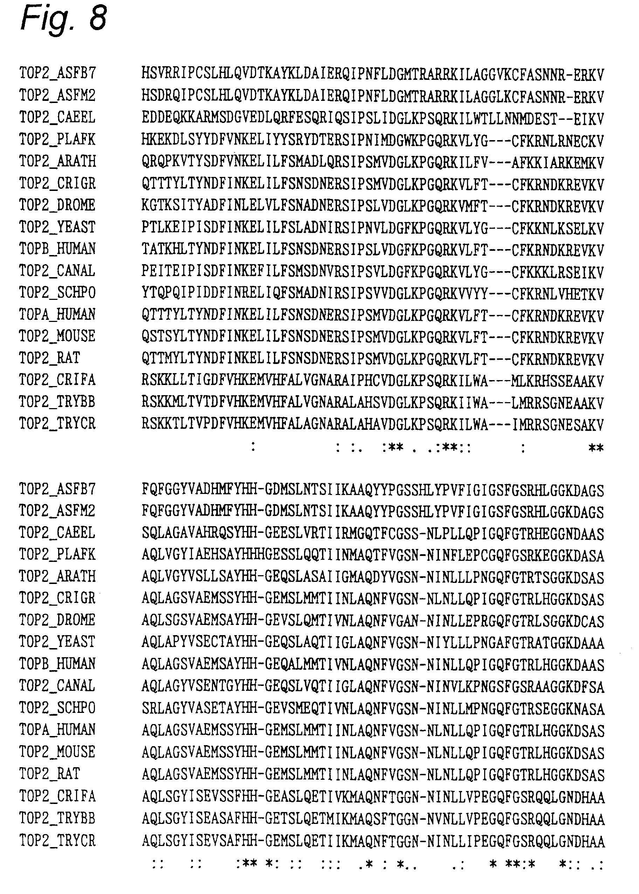

Figs. 1 through 9 show alignment of the amino acid sequences of type II topoisomerase from various eukaryotes. The symbols at the left side in the figure indicate the organisms shown below.

    TOP2_ASFB7: AFRICAN SWINE FEVER VIRUS;
    TOP2_ASFM2: AFRICAN SWINE FEVER VIRUS;
    TOP2_CAEEL: CAENORHABDITIS ELEGANS;
    TOP2_PLAFK: PLASMODIUM FALCIPARUM;
    TOP2_ARATH: ARABIDOPSIS THALIANA;
    TOP2_CRIGR: CRICETULUS GRISEUS;
    TOP2_DROME: DROSOPHILA MELANOGASTER;
    TOP2_YEAST: SACCHAROMYCES CEREVISIAE;
    TOPA_HUMAN, TOPB_HUMAN: HOMO SAPIENS;
    TOP2_CANAL: CANDIDA ALBICANS;
    TOP2_SCHPO: SCHZOSACCHAROMYCES POMBE;
    TOP2_MOUSE: MUS MUSCULUS;
    TOP2_RAT: RATTUS NORVEGICUS;
    TOP2_CRIFA; CRITHIDIA FASCICULATA;
    TOP2_TRYBB: TRYPANOSOMA BRUCEI BRUCEI;
    TOP2_TRYCR: TRYPANOSAMA CRUZI

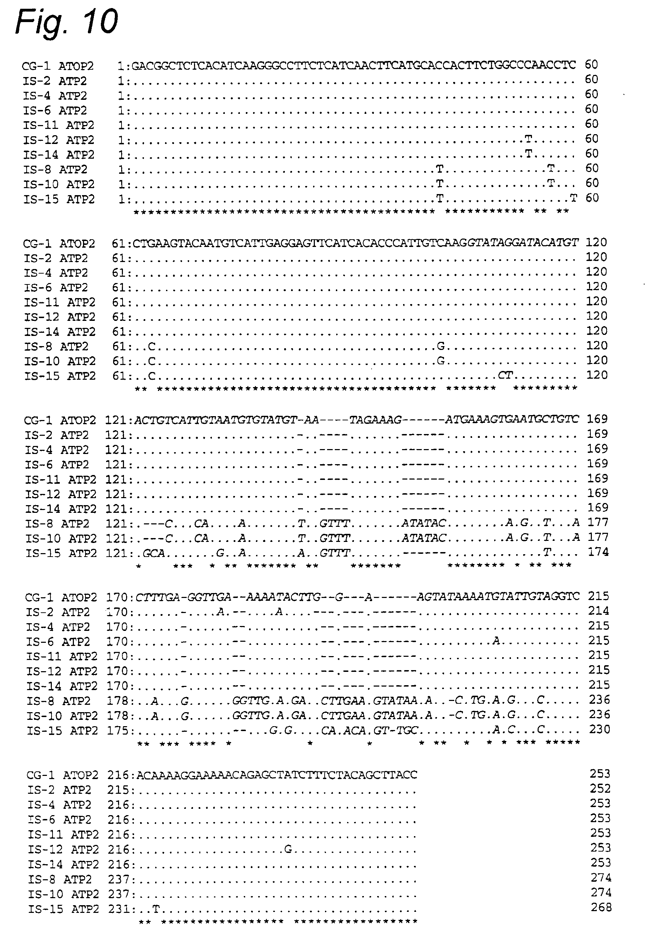

Fig. 10 shows alignment of the nucleic acid base sequences of the intron-containing regions in type II topoisomerases derived from ten individuals of three species belonging to the genus *Crassostrea*. Base sequences shown by italic characters are introns.

DETAILED DESCRIPTION OF THE INVENTION

Hereinafter, the present invention is described in detail.

An identification and detection method according to the present invention is characterized in that it is based on the base sequence of an intron-containing region of a type II topoisomerase gene. The region used for identification and detection is not limited as long as it contains an intron.

The term "identification" means the procedure to affiliate the organism to a group classified based on type II topoisomerase gene sequence; and the term "detection" means the procedure to discover the specific organism in a sample based on type II topoisomerae gene sequence.

Since the sequence of an intron does not have a functional restriction such as the necessity to encode amino acids, it is considered to have undergone more rapid evolution than protein-coding regions. That is, the intron sequence is less conserved (usually, 60% or less). Therefore, it is possible to distinguish among close individuals (for example, strains, lines, growth groups of individuals within the same species may be discriminated) based on the sequence of an intron.

Eukaryotes which can be identified and detected according to the present invention are not particularly limited and include any organisms belonging to Animalia, Plantae, Protista, and Fungi. Examples of preferable eukaryotes include oysters, rice, microalgae and yeasts, but are not limited thereto.

In the practical identification and detection according to the invention, a method taking advantage of the PCR method may preferably be used, in which primers capable of amplifying an intron region are used to carry out PCR with the DNA of a eukaryote sought to be identified. The primers to be used can be suitably synthesized according to the base sequences shown in Figs. 1 through 9 or Examples hereinafter given or known base sequences of type II topoisomerase genes.

Another identification and detection method according to present invention is characterized by comprising amplifying the DNA of a eukaryote by PCR or cDNA cloning using, as at least one of primers or a probe, an oligonucleotide which comprises a base sequence coding for a part of (or the whole of) the following amino acid sequences (a) to (k) and functions as a substantial primer, and identifying the eukaryote based on the base sequence of the amplified DNA:

    (a) Asp-Asp-Glu-Lys-Lys-Val-Thr-Gly
    (b) Lys-Lys-Lys-Asn-Lys-Gly-Gly
    (c) Met-Ile-Met-Thr-Asp-Gln-Asp-Gln-Asp-Gly
    (d) Glu-Glu-Phe-Ile-Thr-Pro-Ile-Val
    (e) Pro-Glu-Phe-Glu-Glu-Trp-Lys
    (f) Arg-Pro-Asp-(Thr or Ser)-Tyr-Ile-Gly
    (g) Leu-(Tyr or Leu or Phe)-Lys-Ile-Phe-Asp-Glu-Ile
    (h) Asn-Gly-(Tyr or Phe)-Gly-Ala-Lys-Leu
    (i) Thr-Ile-Lys-Gly-Gly-Thr-His-Val
    (j) Asp-Ala-Asn-Lys-Ala-Gly-The-Lys
    (k) Val-Asn-Gly-Ala-Glu-Gly-Ile

The amino acid sequences (a) to (k) were selected by comparing the amino acid sequences of type II topoisomerase of organisms belonging to Animalia, Plantae, Protista, and Fungi shown in Figs. 1 through 9; choosing highly conserved amino acid sequences for the respective eukaryotes, and further choosing amino acid sequences having fewer possible degenerate codons by which they are encoded. The relationships between the selected amino acid sequences (a) to (k) and the amino acid sequences of the type II topoisomerase of various eukaryotes are shown in Figs. 1 through 9.

Illustrative examples of base sequences encoding the amino acid sequences (a) to (k) are shown below. Although both of sense strands and antisense strands can be used, example of one of the sense strand and antisense strand is respectively shown below.

    Base sequence corresponding to amino acid sequence (a):
        GAYGAYGARAARAARGTNACNGG (example of sense strand)
    Base sequence corresponding to amino acid sequence (b):
        AARAARAARAAYAARGGNGG (example of sense strand)
    Base sequence corresponding to amino acid sequence (c):
        ATGACNGAYCARGAYCARGAYGG (example of sense strand)
    Base sequence corresponding to amino acid sequence (d):
        GARGARTTYATHACNCCNATHGT (example of sense strand)
    Base sequence corresponding to amino acid sequence (e):
        TTCCAYTCYTCRAAYTCNGG (example of antisense strand)
    Base sequence corresponding to amino acid sequence (f):
        MGNCCNGAYACNTAYATHGG (example of sense strand)
    Base sequence corresponding to amino acid sequence (g):
        YTNTAYAARATHTTYGAYGA (example of sense strand)
    Base sequence corresponding to amino acid sequence (h):

ARYTTNGCNCCRWANCCRTT (example of antisense strand)

Base sequence corresponding to amino acid sequence (i):
ACRTGNGTNCCNCCYTTDATNGT (example of antisense strand)

Base sequence corresponding to amino acid sequence (j);
GAYGCNAAYAARGCNGGNAC (example of antisense strand)

Base sequence corresponding to amino acid sequence (k):
ATNCCYTCNGCNCCRTTNAC (example of sense strand)

The oligonucleotides represented by the above-described base sequences may be used as primers directly, e.g., as they are. Alternatively, if desired, an appropriate sequence can be added thereto. For example, the M13R sequence can be added to the 5'-end of the forward primer, and the M13(-21) sequence to the 5'-end of the reverse primer. By using the sequence of M13R or of M13(-21) as sequence primers, it is possible to directly determine the base sequence of the PCR amplification product without subjecting the PCR product to subcloning. The M13R and M13(-21) sequences are shown below.

M13R sequence:
caggaaacagctatgacc
M13(-21) sequence:
tgtaaaacgacggccagt

The language "an oligonucleotide functioning as a substantial primer or a probe" as used herein means an oligonucleotide whose length is sufficient to hybridize under stringent conditions with a specific region of a template DNA. The actual length of the primer and the probe is preferably from 16-mer to 60-mer, more preferably from 20-mer to 40-mer, and most preferably from 20-mer to 30-mer.

The stringent condition is, for example, as follows. In the case of PCR, Tm is calculated based on the equation $Tm(°C) = 4 \times (G + C) + 2 \times (A + T) - 5$, the temperature is changed around Tm, and the highest temperature at which amplification is possible is employed as the stringent condition. In the case of cDNA cloning, hybridization conditions are, for example, 25°C in 5 X SSC buffer and washing conditions are, for example, 37°C in 2 X SSC buffer.

The present invention is described further in detail below.

The genes for type II topoisomerase are indispensable for the growth of all organisms; therefore, they are suitable for identification and detection of organisms. Because the genes for type II topoisomerase evolved faster than those of ribosomal RNA, which are the standard molecules for those purposes, closely related organisms can be distinguished. The present invention is based on a new finding that an intron-containing sequences in type II topoisomerase gene can be advantageously used for distinguishing the more closely related organisms.

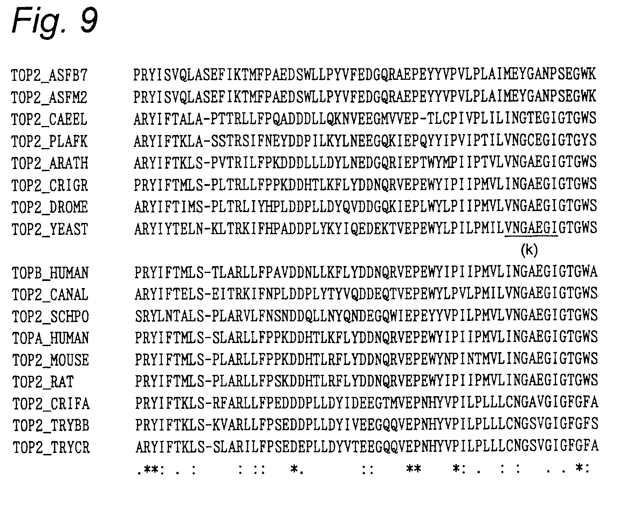

In order to selectively obtain DNA fragments of an intron-containing region of type II topoisomerase gene of one or more species of organisms in a sample, nucleotide sequences of the genomic DNA fragments are determined and the positions of intron are determined as follows. Because it is difficult to speculate as to the positions of intron in advance, it is better to determine the genomic DNA sequence encoding type II topoisomerase gene is so far as possible. In order to obtain the genomic DNA fragment, conserved sequences which can be determied from the reference sequences, which have been already deposited to the public database such as Swiss-Prot protein database, can be utilized. The reference sequences are aligned based on their similarity by using computer program for multiple sequence alignment such as Clustal W (Thompson, J.D., D. G. Higgins, and T. J. Gibson. 1994. Clustal W: improving the sensitivity of progressive multiple sequence alignment through sequence weighting, positions-specific gap penalties and weight matrix choice. Nucleic Acids Res. 22: 4673-4680). The conserved sequences are used as PCR primers and as probes for screening the DNA library in order to obtain the DNA fragment of type II topoisomerase gene. The conserved sequence is preferably 6 or more amino acid sequence having a homology of preferably 80% or more, more preferably 90% or more, still more preferably 95% or more, and most preferably 100%. Several conserved sequences, whose length are more than 6 amino acid residues, were found in the aligned sequences. Then, all possible nucleotide sequences are deduced based on the codon table. Degenerated primers, which are a mixture of all possible nucleotide sequences, can readily be designed based on the following rules. Degenerated sequences, whose numbers of mixture of possible nucleotide sequences are less than 1024, are preferably selected as the primer/probe regions. When a mixture of more than 1024 sequences is required to be selected, their degeneracy (e.g., the number of sequences required) is reduced by using an inosine nucleotide in place of unspecified nucleotides. Nucleotide sequences having higher homology at 3'-side are preferable for the PCR primers. Several regions were found as universal primers, for example, sequence numbers (a) to (k). In them, combinations of sense strand sequences of either sequence number (f) or (g) in Fig. 1, and anti-sense strand sequence of sequence number (k) in Fig. 9 are particularly useful to obtain the nearly whole genomic sequence of type II topoisomerase gene. However, when the amplified fragment is too long for PCR, both sense and antisense strands sequences in the internal regions, i.e. sequence numbers (a) to (e) and (h) to (j) are useful to obtain the genomic sequence. The length of the internal regions is not particularly limited and preferably around 1 kb. However, the regions longer than 1 kb can be used by utilizing, for example, long PCR protocols described by Barnes (Barnes, W. M. 1994. PCR amplification of up to 35-kb DNA with high fidelity and high yield from lambda bacteriophage templates. *Proc. Natl. Acad. Sci*. *USA* 91: 2216-20) are also used to amplify the long stretch of the genomic DNA. When the amplified fragments are directly used for the determination of nucleotide sequence, the specified sequence, such as either M13R, M13 (-21), T7, T3 or SP6 sequence (preferably from 15 to 20 bases), may be further added at the 3' -end of the sequence, which anneals to the genomic DNA encoding type II topoisomerase. All the oligonucleotides can be chemically synthesized. When the conserved sequences are used as probes, both strands sequences are useful for all regions. Nucleotide sequences of the resultant amplified fragment or clones are determined and translated in three phases. Deduced amino acid sequences are compared with a plurality of reference sequences of type II topoisomerase. Among conserved sequences, combinations of two conserved sequences that can amplify an intron-containing sequence are selected. In many cases in which intron exist in the determined sequences, sequence stretch, which is similar to reference sequences, suddenly interrupted by the insertion sequence. When the consensus sequences of 5'- and 3'- ends of intron, i.e. 5'-GT...AG-3', are found at the region, the region can be regarded as the probable intron. Of course, in order to confirm the existence of the intron, the cDNA fragment should be obtained by RT-PCR or by cDNA-cloning and determined the nucleotide sequence of the cDNA fragment.

Then, the intron-containing sequences in type II topoisomerase genes of various organisms are accumulated to provide a database.

In order to achieve the identification of the strains by using the intron-containing sequence, the intron-containing sequence of the strain should be compared with a plurality of reference sequences or in the database constructed as described above. Conserved sequences are found in the flanking regions of the intron and are used as primers or probes to obtain the intron-containing DNA fragment from the different individual strains. To search the conserved sequences, sequences of intron-containing DNA fragment are aligned based on their similarity by using computer program for multiple sequence alignment such as Clustal W (Thompson, J.D., D. G. Higgins, and T. J. Gibson. 1994. Clustal W: improving the sensitivity of progressive multiple sequence alignment through sequence weighting, positions-specific gap penalties and weight matrix choice. *Nucleic Acids Res*. 22: 4673-4680). In the exon region, conserved sequences, whose length are more than 6 amino acid residues, are found in the aligned sequences, and all possible nucleotide sequences of the regions are speculated. Degenerated sequences, whose numbers of mixture of possible nucleotide sequences are less than 1024, are preferably selected and used as the PCR primers for amplification of the intron-containing DNA fragment. When degenerated sequences, whose numbers of mixture of possible nucleotide sequences are greater than 1024 need to be selected, their degeneracy (e.g., the number of possible sequences) is reduced by using an inosine nucleotide in place of unspecified nucleotides. All oligonucleotides for primers can be chemically synthesized. When the amplified fragments are directly used to determine nucleotide sequence, the specified sequence, such as either M13R, M13 (-21), T7, T3 or SP6 sequence, is added at the 3'-end of the primers, which anneal the DNA segment encoding the type II topoisomerase. The intron sequences of the strain are compared with a plurality of reference sequences or in the database by using a computer program such as Blast (Altschul, S. F., Gish, W., Miller, W., Myers, E. W. & Lipman, D. J. 1990. Basic local alignment search tool. *J. Mol. Biol.* 215: 403-410). When completely matched sequence is found in plural reference sequences, the strain is identified as that reference strain. When the sequence does not match to any reference sequences or in the database, closely related strains are found in reference strains by phylogenetic analysis by using program packages such as Clustal W (Thompson, J.D., D. G. Higgins, and T. J. Gibson. 1994. Clustal W: improving the sensitivity of progressive multiple sequence alignment through sequence weighting, positions-specific gap penalties and weight matrix choice. Nucleic Acids Res. 22: 4673-4680), or Phylip (Felsenstein, J. 1993. PHYLIP (Phylogeny Inference Package) version 3.5c. Distributed by the author, Department of Genetics, University of Washington, Seattle, U.S.A.).

In the present invention, the detection of the strains means that DNA segments of an intron-containing region in type II topoisomerase of one or more strains are specifically found in a sample. To achieve the detection of the strain, it is preferable to find the specific features in the intron-containing region in type II topoisomerase of one or more strains. "Specific features" of the intron-containing region include differences in nucleotide sequence of the intron, length of the intron, position of the intron, etc. Such specific features are found by comparison of the nucleotide sequences of an intron-containing region between one strain and other closely related strains. To obtain the intron-containing DNA fragment from the strains, conserved sequences are found in the flanking regions of the intron. The sequences of the intron-containing DNA fragment are aligned based on their similarity by using computer program for multiple sequence alignment such as Clustal W (Thompson, J.D., D. G. Higgins, and T. J. Gibson. 1994. Clustal W: improving the sensitivity of progressive multiple sequence alignment through sequence weighting, positions-specific gap penalties and weight matrix choice. *Nucleic Acids Res*. 22: 4673-4680). Conserved sequences, whose length are more than 6 amino acid residues, were found in the aligned sequences, and all possible nucleotide sequences of the regions are speculated. As before, sequences, whose numbers of mixture of possible nucleotide sequences are less than 1024, are preferably selected and used as the PCR primers for amplification of the intron-containing DNA fragment and the degeneracy of sequences whose numbers of mixture are more than 1024, is preferably reduced by using an inosine nucleotide in place of unspecified nucleotides. All oligonucleotides for primers can be chemically synthesized. When the amplified fragments are directly used for determination of the nucleotide sequence, the specified sequence, such as either M13R, M13 (-21), T7, T3 or SP6 sequence, is added at the 3'-end of the primers, which anneal the DNA segment encoding the type II topoisomerase. By using the oligonucleotide primers, the intron-containing DNA fragments from the closely related strains, are amplified and determined the nucleotide sequences. The resultant sequences are compared by using computer programs such as Blast (Altschul, S. F., Gish, W., Miller, W., Myers, E. W. & Lipman, D. J. 1990. Basic local alignment search tool. *J. Mol. Biol.* 215: 403-410), or Clustal W (Thompson, J.D., D. G. Higgins, and T. J. Gibson. 1994. Clustal W: improving the sensitivity of progressive multiple sequence alignment through sequence weighting, positions-specific gap penalties and weight matrix choice. *Nucleic Acids Res*. 22: 4673-4680).

The parameters for the aforementioned alignment by the computer program Clustal W are, for example, as follows.

1. Gap Open Penalty: 10.00
2. Gap Extension Penalty: 0.10
3. Protein Weight Matrix: BLOSUM 30
4. DNA Weight Matrix: IUB

The parameters for the aforementioned phylogenetic analysis by the computer program Phylip are, for example, as follows. Genetic distance matrices based on Kimura's 2-parameter model (Kimura, M. (1980) *J. Mol. Evol*. 16, 111-120) with the transition/transversion ratio of 2.0 are produced by using the DNADIST program, and the neighbor-joining tree is constructed by using the NEIBHBOR program with selecting "neighbor-joining" for assumption. Both programs are packaged in Phylip.

In addition, the identification and detection based on the intron-containing genomic DNA the specific strain can be achieved also by any suitable or convenient molecular biological technique, such as strain-specific PCR, PCR-RFLP, PCR-SSCP, quantitative PCR, dot hybridization, DNA chip, etc. For some methods, primers or probes may desirably labeled with isotope or fluorescence for even quicker identification.

EXAMPLE 1

Identification of oyster using type II topoisomerase gene:

(1) Preparation of oyster chromosomal DNA

DNAs were prepared from samples of an oyster individual and of other organisms in accordance with DNA preparation techniques suited to the respective organisms. The DNA thus prepared was diluted with a TE buffer to 0.05 μg/μl and kept at 4°C before the use a template DNA for PCR.

(2) PCR and sequence determination of amplified DNA of oyster type II topoisomerase gene

PCR was carried out using one of three DNA polymerase enzymes; AmpliTaq Gold and rTth (both produced by Perkin-Elmer) and LaTaq polymerase (produced by Takara Shuzo). As the buffers, etc., those included in the kits were used with the concentrations specified by the suppliers. PCR primers were both used in a concentration of 2 μM. The primers having the following base sequences were used. The sequences written in capital letters correspond to the specific amino acid sequence selected, and those in small letters are of sequence primers M13R and M13(-21).

        Forward side primer: ATOP2-526
            caggaaacagctatgaccATGACNGAYCARGAYCARGAYGG
        Reverse side primer: ATOP2-573R
            tgtaaaacgacggccagtTTCCAYTCYTCRAAYTCNGG

These primers correspond to the amino acid sequences (c) and (e), respectively. The PCR was carried our for 40 cycles, each cycle consisting of alternating temperatures of 95°C (denaturation), 57°C (annealing), and 72°C (extension) for 1 minute, 30 seconds, and 2 minutes, respectively, in a Progene type thermal cycler (Techne Co.). The amplified products were subjected to electrophoresis on a low melting point agarose gel, so that the desired amplified DNA products were separated from unreacted primers and recovered by use of QIAquick Gel Extraction Kit (Qiagen Co.). The sequence of the amplified DNA fragment thus obtained was determined using the amplified fragment as a template and the sequences of M13R and of M13(-21) as sequence primers. The sequence reaction was carried out by means of Big Dye Terminator Cycle Sequencing FS Ready Reaction Kit (PE Applied Biosystems). The reaction product was analyzed with 377 type DNA Sequencer (PE Applied Biosystems) to determine the base sequence.

The sequences of the amplified DNA of *Crassostrea gigas* and *Crassostrea lurida* and the amino acid sequences based thereon are shown below. The regions showing the base sequence without corresponding amino acids are intron sequences.

```
       Origin: Crassostrea gigas; Collected in Matsushima Bay,
       Miyagi Prefecture, Japan

       Length of Sequence: 252

               10        20        30        40        50        60
       GACGGCTCTCACATCAAGGGCCTTCTCATCAACTTCATGCACCACTTCTGGCCCAACCTC
        D   G   S   H   I   K   G   L   L   I   N   F   M   H   H   F   W   P   N   L
               70        80        90        100       110       120
       CTGAAGTACAATGTCATTGAGGAGTTCATCACACCCATTGTCAAGGTATAGGATACATGT
        L   K   Y   N   V   I   E   E   F   I   T   P   I   V   K
               130       140       150       160       170       180
       ACTGTCATTGTAATGTGTATGTAATAGAAAGATGAAAGTGAATGCTGTCCTTTGAGGTTA

               190       200       210       220       230       240
       AAAAAAACTTGAAGTATAAAATGTATTGTAGGTCACAAAAGGAAAAACAGAGCTATCTTT
                                          V   T   K   G   K   T   E   L   S   F
               250
       CTACAGCTTACC
        Y   S   L
```

Origin: Crassostrea lurida; collected on the west coast of North America

Length of Sequence: 1014

```
          10        20        30        40        50        60
GACGGATCTCACATCAAGGTTCTTCTGCTTAACTTCATGCATCATTTCTGGCCCAACTTG
 D   G   S   H   I   K   V   L   L   L   N   F   M   H   H   F   W   P   N   L

          70        80        90       100       110       120
TTGAAACACAATGTTGTCAACCAGTTTATTACACCCATTGTCAAGGTTAGTTAAAGGGTA
 L   K   H   N   V   V   N   Q   F   I   T   P   I   V   K

         130       140       150       160       170       180
CCAGTTTATGACTCGCATTGTCGATGATATGTATTGGGTACCAGTTTATGACATTCATTG

         190       200       210       220       230       240
TCAAAATTAGTTACAGGGTACCAGTTTAATACACCCATTGTCAAGGTTAGTTATTAGGTA

         250       260       270       280       290       300
CCAGTTTAAAATGCCAATTATCAAGGTTATTTATAGAATACCAGTTTCTTTTCTCCCATT

         310       320       330       340       350       360
GTCAATTTTATGTTTTAAAGAGCCATTCTTTTGAATGTAATATTAAAATTACTTCATTAT

         370       380       390       400       410       420
CAATAACATTGGAGCTAAAATTACTTCATTATCAACAATATTGGAGCTAAAATTACTTCA

         430       440       450       460       470       480
TTATCAACAACATCGGAGCAAAAATTACTTCATTATCAACAATATTGGAGCTAAAATTAC

         490       500       510       520       530       540
TTCATTATAAACAATATTGGAGCTAAAATTACTTCATTATTAACAACATTGGAGCTAAAA

         550       560       570       580       590       600
TTACTTCATTATCAACAACATTGGAGCTACGGTGACTGAGTGATCAGCCTTTGTTCTATG

         610       620       630       640       650       660
CTGTCACAATGGAGTATGAAGCTTGGACCTTTGACTAAATACTATTTAAGCTTAATGCAA

         670       680       690       700       710       720
GTCAGTAGCCTACTGATGGTTTTTCTCCAGTTATGTCTTTAAAGATTAATAAGGTCAAAG

         730       740       750       760       770       780
AGGAACTGTCAATTTACAGTTTATATATATATTTATAGGTAACTAAGGTCAAAGAGGAAC

         790       800       810       820       830       840
AATCATTTTACAGTTTTATATATCTTTATAGGTAACTAAGGTCAAAGAGGAACTGTCATT
```

9

```
        850       860       870       880       890       900
TTACAGTTTATATATATCTTTATAGGTAACTAAGGGCAAAGAAGAACTGTCATTTTACAG

        910       920       930       940       950       960
TTTATATATATCTTTATAGGTAACTAAGGGCAAAGAGGAACTGTCATTTTACAGTTTATA

        970       980       990      1000      1010      1020
TATATCTTTATAGGTAACTAAGGGCAAGGAAGAACTGTCATTTTACAGTTTACC
         V   T   K   G   K   E   E   L   S   F   Y   S   L
```

(3) Study of other primers for amplification of oyster type II topoisomerase gene

In the same manner as described above, the type II topoisomerase gene fragment was amplified by PCR using other sets of primers; a set of primers comprising base sequences coding for the amino acid sequences (a) and (d); a set of primers comprising base sequences coding for the amino acid sequences (b) and (e); a set of primers comprising base sequences coding for the amino acid sequences (b) and (d); and a set of primers comprising base sequences coding for the amino acid sequences (b) and (c). The following primers corresponding to the amino acid sequences (a), (b), and (d) were used. The sequences written in capital letters correspond to the specific amino acid sequence selected, and those in small letters are of sequence primers M13R and M13(-21).

     Primer corresponding to the amino acid sequence (a) (ATOP2-141F):
          caggaaacagctatgaccGAYGAYGARAARAARGTNACNGG
     Primer corresponding to the amino acid sequence (b) (ATOP2-329F):
          caggaaacagctatgaccAARAARAARAAYAARGGNGG
     Primer corresponding to the amino acid sequence (d) (ATOP2-551R):
          tgtaaaacgacggccagtACDATNGGNGTDATRAAYTCYTC

As a result, it was confirmed that amplification of the type II topoisomerase gene fragment is possible with any of these sets of primers. It is believed that the amplified fragment contained an intron region in each case. (4) Studies on the sequence diversity of the oyster type II topoisomerase genes among genera, species and individuals

Comparison among amino acid sequences translated from the resulting base sequences revealed that there is no amino acid substitution among the genus *Crassostrea*, indicating that all the nucleic acid base substitution is a degenerate neutral mutation. On the other hand, considerable amino acid substitution was observed between different genera, i.e., the genus *Ostrea* and the genus *Crassosterea*. Amino acid substitution was also found within the genus *Ostrea*. From these results, it is understood that the species belonging to different genera or the species belonging to the same genus but having a phylogenetically distance from each other can be distinguished based on their amino acid sequences or DNA sequences encoding the same.

Fig. 10 shows alignment of the nucleic acid base sequences of the intron-containing regions in type II topoisomerases derived from ten individuals of three species belonging to the genus *Crassostrea*, which were amplified by a set of a primer comprising the sequence coding for the amino acid sequence (c) and a primer comprising the sequence coding for the amino acid sequence (e). Among closely-related species, homology is observed even in the intron-containing regions to some extent. Further, substitution or a gap is observed in the intron sequence among individuals of the same species, which apparently suggests that the intron region has been evolving more rapidly than protein-coding regions. As a result of phylogenetic analysis based on the intron sequences, it was possible to classify *Crassostrea gigas* into 6 lines. It is therefore thought that the type II topoisomerases are useful in distinguishing individuals more finely based on, for example, differences in strain or line or growth group among individuals of the same species.

EXAMPLE 2

Identification of eukaryotes other than oysters using type II topoisomerase gene:

The sequences of type II topoisomerase genes of other eukaryotes which were obtained by using primers corresponding to the amino acid sequences (a) to (k) are shown below. Existence of an intron was also confirmed in a unicellular red alga and a basidiomycetous yeast. DNA amplification of the type II topoisomerase gene fragment was achieved by means of a set of primers comprising the sequences coding for the amino acid sequences (f) and (h), or (g) and (i) as for the unicellular alga and rice; a set of primers comprising the sequences coding for the amino acid sequences (f) and (j), or (j) and (k) as for an ascomycetous yeast; and a set of primers comprising the sequences coding for the amino acid sequences (g) and (h) as for the basidiomycetous yeast.

The following primers which correspond to the amino acid sequences (f), (g), (h), (i), (j), and (k) were used.

Primer comprising base sequence coding for amino acid sequence (f) (TOP2-53F):
      caggaaacagctatgaccMGNCCNGAYACNTAYATHGG
Primer corresponding to the amino acid sequence (g) (TOP2-87F):
      caggaaacagctatgaccYTNTAYAARATHTTYGAYGA
Primer corresponding to the amino acid sequence (h) (TOP2-163R):
      tgtaaaacgacggccagtARYTTNGCNCCRWANCCRTT
Primer corresponding to the amino acid sequence (i) (TOP2-322R):
      tgtaaaacgacggccagtACRTGNGTNCCNCCYTTDATNGT
Primer corresponding to the amino acid sequence (j):

      Primer paired with (f) (TOP2-431R): antisense
            tgtaaaacgacggccagtNCCNGCYTTRTTNGCRTC
      Primer paired with (k) (YTOP2-437F): sense
            caggaaacagctatgaccGAYGCNAAYAARGCNGGNAC

Primer corresponding to the amino acid sequence (k) (YTOP2-826R):
      tgtaaaacgacggccagtATNCCYTCNGCNCCRTTNAC

(i) unicellular alga
Origin: Rhodosorus sp. 721-C

Length of Sequence: 327

```
        10        20        30        40        50        60
TCTGCTGAGAAGACAACAATACCACTATGGCTCTGGAACGACGAGGAGGAATGCATGGAG
 S   A   E   K   T   T   I   P   L   W   L   W   N   D   E   E   E   C   M   E

        70        80        90       100       110       120
CATCGCGAAGCGTCTTTCGTTCCGGGGCTTTACAAAATCTTCGACGAAATTCTAGTAAAT
 H   R   E   A   S   F   V   P   G   L   Y   K   I   F   D   E   I   L   V   N

       130       140       150       160       170       180
GCGGCGGATCATCGGCAGAGGGATTCGTCCATGGATACCATTGAAGTGTTCATTGACGAA
 A   A   D   H   R   Q   R   D   S   S   M   D   T   I   E   V   F   I   D   E

       190       200       210       220       230       240
GAGAACAACCGAATTTCTGTGAAAAACAATGGAAGTGGTATTCCGGTTGAGGTTCATAAA
 E   N   N   R   I   S   V   K   N   N   G   S   G   I   P   V   E   V   H   K

       250       260       270       280       290       300
AAAGAAGGAGTTTACGTACCAGAGCTCATCTTCGGGCATTTGCTCACCTCTTCGAATTAC
 K   E   G   V   Y   V   P   E   L   I   F   G   H   L   L   T   S   S   N   Y

       310       320       330
AATGACAATGAGAAAAAAGTAGTTGGA
 N   D   N   E   K   K   V   V   G
```

(ii) unicellular alga
Origin: Galdieria sulphuraria NIES250

Length of Sequence : 382

```
       10        20        30        40        50        60
TCGGTGGAACGAACGACCCAAACTTTATGGGTATACGATAGCGTAAATCAACGAATGACC
 S   V   E   R   T   T   Q   T   L   W   V   Y   D   S   V   N   Q   R   M   T

       70        80        90       100       110       120
TTTCGTGAAGTGTCATTCGTCCCTGCGCTATACAAAATCTTCGATGAAATTTTAGTAAAT
 F   R   E   V   S   F   V   P   A   L   Y   K   I   F   D   E   I   L   V   N

      130       140       150       160       170       180
GCAGCTGACCATAAGCAGCGTGATCCTTCTATGAATACGATACGTGTTGACATTCACAAG
 A   A   D   H   K   Q   R   D   P   S   M   N   T   I   R   V   D   I   H   K

      190       200       210       220       230       240
GAGGATAACAAGATATCCATATATAACAACGGAAAGGGTATTCCTGTCGAGGTAAGGGAT
 E   D   N   K   I   S   I   Y   N   N   G   K   G   I   P   V   E

      250       260       270       280       290       300
ACAAGTAAAAATATTACAACTGCTAAAAACATCGGCTATATTGCAGATACACAAAAAAGA
                                                         I   H   K   K   E

      310       320       330       340       350       360
AAAAATCTATGTACCGGAAATGATTTTCGGCCATCTTCTTACGTCATCTAACTATGATGA
 K   I   Y   V   P   E   M   I   F   G   H   L   L   T   S   S   N   Y   D   D

      370       380       390
CAGCGAAAGGAAAGTTGTTGGA
 S   E   R   K   V   V   G
```


(iii) unicellular alga
Origin: Porphridium aeruginum UTEX755

Length of Sequence: 327

```
       10        20        30        40        50        60
TCTGCAGAGAGAATTACTCAAACCATGTGGGTATGGGATGAACTTGCGGGTAAGATGGTT
 S   A   E   R   I   T   Q   T   M   W   V   W   D   E   L   A   G   K   M   V

       70        80        90       100       110       120
TATCGCGATGTGTCCTATGTACCGGGTCTCTACAAAATCTTTGACGAGATTTTGGTGAAT
 Y   R   D   V   S   Y   V   P   G   L   Y   K   I   F   D   E   I   L   V   N
```

```
              130       140       150       160       170       180
GCGGCGGACCACAAGCAGCGAGACGCATCTTTGGACACCATTGAAGTGAACATTGACGTG
A    A    D    H    K    Q    R    D    A    S    L    D    T    I    E    V    N    I    D    V

              190       200       210       220       230       240
GAGAACAACTCCATTTCAGTATACAACAACGGAAATGGTATTCCTGTGGAGATCCACAAG
E    N    N    S    I    S    V    Y    N    N    G    N    G    I    P    V    E    I    H    K

              250       260       270       280       290       300
AAGGAAGGGGTTTACGTCCCGGAGATGATCTTCGGCCAGTTGCTGACCTCGTCCAACTAT
K    E    G    V    Y    V    P    E    M    I    F    G    Q    L    L    T    S    S    N    Y

              310       320       330
AACGACGATGAAAAGAAGGTGGTGGGC
N    D    D    E    K    K    V    V    G
```

(iv) rice
Origin: Oryza sativa

Variety: Yamada Nishiki

Length of Sequence: 318

```
              10        20        30        40        50        60
TCGGTGGAGAAGCACACCGCGCAGCTCTGGGTGTACGAGGACGGCGCCATGGTGAGCCGC
S    V    E    K    H    T    A    Q    L    W    V    Y    E    D    G    A    M    V    S    R

              70        80        90        100       110       120
AGCGTCACCTACGTCCCCGGCCTCTACAAGATCTTCGACGAGATACTCGTCAACGCCGCC
S    V    T    Y    V    P    G    L    Y    K    I    F    D    E    I    L    V    N    A    A

              130       140       150       160       170       180
GACAACAAGCAGCGCGACCCCTCCATGGACTCCCTCCGCGTCGAGATCGACGCCGACGAG
D    N    K    Q    R    D    P    S    M    D    S    L    R    V    E    I    D    A    D    E

              190       200       210       220       230       240
GGCCGCATCTCCGTCTACAACAACGGCGACGGCATCCCCGTCGAGATCCACCAGGAGGAG
G    R    I    S    V    Y    N    N    G    D    G    I    P    V    E    I    H    Q    E    E

              250       260       270       280       290       300
GGCGTTTACGTGCCGGAGATGATATTCGGCCACCTCCTCACCAGTAGCAATTACGACGAC
G    V    Y    V    P    E    M    I    F    G    H    L    L    T    S    S    N    Y    D    D

              310       320
AACGTGAAGAAGACCACC
N    V    K    K    T    T
```

(v) ascomycetous yeast
Origin: Candida zeylanoides JCM 1627T

Length of sequence: 1269

```
        10        20        30        40        50        60
TCGGTCGAAAGATCCACGCAGGAGATGTGGGTCTACAATGCCGAAACTGATGCGATGGCT
 S   V   E   R   S   T   Q   E   M   W   V   Y   N   A   E   T   D   A   M   A

        70        80        90        100       110       120
TTCAGTGAAGTCACGATTGTGCCCGGACTTTACAAGATCTTTGACGAGATCTTGGTCAAT
 F   S   E   V   T   I   V   P   G   L   Y   K   I   F   D   E   I   L   V   N

        130       140       150       160       170       180
GCCGCCGACAACAAGATCAGAGACCCACAGATGAGAAATATTCGTGTGTCCATCGATGCC
 A   A   D   N   K   I   R   D   P   Q   M   R   N   I   R   V   S   I   D   A

        190       200       210       220       230       240
GAAGCAAACACTATTGAGGTGATGAATGATGGACGTGGGATACCGGTGGAGATCCACGAC
 E   A   N   T   I   E   V   M   N   D   G   R   G   I   P   V   E   I   H   D

        250       260       270       280       290       300
AAGGAGCAAATGTACATTCCTGAATTGATTTTCGGGAACTTGTTGACGTCTTCTAACTAT
 K   E   Q   M   Y   I   P   E   L   I   F   G   N   L   L   T   S   S   N   Y

        310       320       330       340       350       360
GATGACGATCAACGAAAAGTCACTGGCGGGGCGCAACGGGTTTGGCGCCAAGTTGTGTAAC
 D   D   D   Q   R   K   V   T   G   G   R   N   G   F   G   A   K   L   C   N

        370       380       390       400       410       420
ATTTTCTCAACGGAATTTGTTGTGGAGACTGCTGACTTGAGCAATGAAAAATTGTACCGC
 I   F   S   T   E   F   V   V   E   T   A   D   L   S   N   E   K   L   Y   R

        430       440       450       460       470       480
CAAGTGTGGACCTCCAACATGTCGACGGTGGGCAAGCCCAAGATCACCAAGATGAAGAGC
 Q   V   W   T   S   N   M   S   T   V   G   K   P   K   I   T   K   M   K   S

        490       500       510       520       530       540
AAACGTGAATTCACAAAAATCACCTTCAAGCCGGACTTGGCCAAATTTGGGATGGAAAGG
 K   R   E   F   T   K   I   T   F   K   P   D   L   A   K   F   G   M   E   R

        550       560       570       580       590       600
TTAGACCCAGAGATCTTGAGCGTTATGCGAAGAAGGGTGTATGACTTATGTGGCACCGTC
 L   D   P   E   I   L   S   V   M   R   R   R   V   Y   D   L   C   G   T   V

        610       620       630       640       650       660
AAAGATTGCAATGTCTACTTGAATGATAAGAAGTTGCCCGTGAAAAATTTCAAGGGGTAT
 K   D   C   N   V   Y   L   N   D   K   K   L   P   V   K   N   F   K   G   Y
```

15

```
          670        680        690        700        710        720
GTCGAGATGTACGTCAAGGCGATGGCATCAAAGGATCAGCGTGCTGTTTCAGATGTACAA
V   E   M   Y   V   K   A   M   A   S   K   D   Q   R   A   V   S   D   V   Q
```

```
          730        740        750        760        770        780
GATCCCATCAAAAGTGAGCCGCTGCTGGAACTTGCCATAGTGTCACCTCCCGTGATCCAA
D   P   I   K   S   E   P   L   L   E   L   A   I   V   S   P   P   V   I   Q
```

```
          790        800        810        820        830        840
AAGGCTCCTCCGATCATCCATGAGGTGCTCAACGACCGCTGGGAGCTTGCATTTGCGGTC
K   A   P   P   I   I   H   E   V   L   N   D   R   W   E   L   A   F   A   V
```

```
          850        860        870        880        890        900
TCCGATGGCTCATTCAATAAAATCTCATTTGTCAATTCCATTGCAACCACATCCGGAGGC
S   D   G   S   F   N   K   I   S   F   V   N   S   I   A   T   T   S   G   G
```

```
          910        920        930        940        950        960
ACTCATGTCAAGTATGTGTCTGACCAAGTTATTAACAAACTCGTGGAAACCTTGAACAAG
T   H   V   K   Y   V   S   D   Q   V   I   N   K   L   V   E   T   L   N   K
```

```
          970        980        990       1000       1010       1020
AGATTGAAAGGCGGCAAGAACAAAAAACTCTTGATCAAGCCTGCTGAGGTGCGTAATCAC
R   L   K   G   G   K   N   K   K   L   L   I   K   P   A   E   V   R   N   H
```

```
         1030       1040       1050       1060       1070       1080
CTTTTTGTTTTCATTAACTGTCTTATCGAGAACCCTGCCTTCACTTCGCAGACAAAGGAG
L   F   V   F   I   N   C   L   I   E   N   P   A   F   T   S   Q   T   K   E
```

```
         1090       1100       1110       1120       1130       1140
CAGCTCACAACAAAGGTCGCCCAATTCGGCAGCAAAGTTGTGATTAGTGACCAGTTCATC
Q   L   T   T   K   V   A   Q   F   G   S   K   V   V   I   S   D   Q   F   I
```

```
         1150       1160       1170       1180       1190       1200
AATAAGATTATCAAATCAACGAATATTGAAGATAAGTTGTTAGAGATAGCTGGGATCAAT
N   K   I   I   K   S   T   N   I   E   D   K   L   L   E   I   A   G   I   N
```

```
         1210       1220       1230       1240       1250       1260
GAAGATAAGCAGTTACAAAAGGCCGATGGTTCCAAAAAGCAAAGGATCAAAGGCCAAGTT
E   D   K   Q   L   Q   K   A   D   G   S   K   K   Q   R   I   K   G   Q   V
```

```
         1270
AAGTTGGTG
K   L   V
```

(vi) ascomycetous yeast
Origin: Candida parapsilosis JCM 1785T

Length of sequence: 1188

```
          10        20       . 30        40        50        60
AAGGCGGGGACGAGAGATGGATTAAAGTGCACTTTGATCCTTACCGAAGGGTTGTCGGCT
 K   A   G   T   R   D   G   L   K   C   T   L   I   L   T   E   G   L   S   A

          70        80        90       100       110       120
TTAAACTTGGCGGTTGCTGGGCTAACTGTTATCGGAAGAGATTACTATGGCTGCTTTCCG
 L   N   L   A   V   A   G   L   T   V   I   G   R   D   Y   Y   G   C   F   P

         130       140       150       160       170       180
TTGAGAGGAAAATTGTTGAACGTTCGTGAAGCTTCCGCTGATCAAATTGCAAAGAACGCT
 L   R   G   K   L   L   N   V   R   E   A   S   A   D   Q   I   A   K   N   A

         190       200       210       220       230       240
GAAATTAATTCATTGAAACAAATTATTGGTCTTCAGCATAAAAAGACTTACAATGCGGAG
 E   I   N   S   L   K   Q   I   I   G   L   Q   H   K   K   T   Y   N   A   E

         250       260       270       280       290       300
AACATCAAGTCGTTGAGGTATGGGCATATTATGATTATGACAGATCAAGATCAAGATGGA
 N   I   K   S   L   R   Y   G   H   I   M   I   M   T   D   Q   D   Q   D   G

         310       320       330       340       350       360
TCACATATTAAAGGTTTGATTATCAATTTTTTGGAAACTTCGTTTCCTGGGTTGCTTGAT
 S   H   I   K   G   L   I   I   N   F   L   E   T   S   F   P   G   L   L   D

         370       380       390       400       410       420
ATTCCTGGGTTTTTGCTTGAATTCATTACACCTATTGTCAAAGTCACTGTGAAAGGTCGT
 I   P   G   F   L   L   E   F   I   T   P   I   V   K   V   T   V   K   G   R

         430       440       450       460       470       480
GGGGCCAAAAAGATAATTCCATTTTACAGCATGCCAGAATTTGAAACATGGAGAGATGGT
 G   A   K   K   I   I   P   F   Y   S   M   P   E   F   E   T   W   R   D   G

         490       500       510       520       530       540
GAAGGTCAAACTTGTCGTTGGACTCAAAAGTACTACAAGGGTTTGGGTACTTCAACTCCC
 E   G   Q   T   C   R   W   T   Q   K   Y   Y   K   G   L   G   T   S   T   P

         550       560       570       580       590       600
ATGGAAGCTCGAGAATATTTTACAGCCTTGGATAGACACTTGAAAAGATTCCATGCATTG
 M   E   A   R   E   Y   F   T   A   L   D   R   H   L   K   R   F   H   A   L

         610       620       630       640       650       660
CAAGGTGAGGATAGTAGCTGTATTGACCTAGCATTCTCCAAAAAGAAGGCTGATGAAAGA
 Q   G   E   D   S   S   C   I   D   L   A   F   S   K   K   A   D   E   R

         670       680       690       700       710       720
AAGGAATGGTTGCAAAATTTTGTTCCTGGTAATCAACTAGATCCCGCGTTGAATGAAATA
 K   E   W   L   Q   N   F   V   P   G   N   Q   L   D   P   A   L   N   E   I

         730       740       750       760       770       780
CCCATAAGTGAATTTATAAACAAGGAATTGATTTTATTTTCGATGTCGGATAACATTAGA
 P   I   S   E   F   I   N   K   E   L   I   L   F   S   M   S   D   N   I   R
```

```
           790        800        810        820        830        840
TCTATCCCTTCTGTGCTTGATGGGCTAAAGCCAGGTCAAAGAAAAGTCTTGTTTGGTTGT
 S   I   P   S   V   L   D   G   L   K   P   G   Q   R   K   V   L   F   G   C
```

```
           850        860        870        880        890        900
TTCAAGAGAAATTTGAAAAGTGAAATCAAAGTGGCACAATTGGCAGCGTATGTAAGTGAA
 F   K   R   N   L   K   S   E   I   K   V   A   Q   L   A   A   Y   V   S   E
```

```
           910        920        930        940        950        960
AATACTGGCTACCATCATGGTGAAGTCTCCTTGGTGCAAACAATCATTGGTCTTGCACAA
 N   T   G   Y   H   H   G   E   V   S   L   V   Q   T   I   I   G   L   A   Q
```

```
           970        980        990       1000       1010       1020
AACTTTGTTGGGTCAAACAACATCAACATTTTGAAACCAAATGGTGCTTTCGGTTCGAGA
 N   F   V   G   S   N   N   I   N   I   L   K   P   N   G   A   F   G   S   R
```

```
          1030       1040       1050       1060       1070       1080
GCAACTGGTGGTAAAGATGCCGCTGCTGCGAGATATATCTTTACTGAGTTGAATGATATA
 A   T   G   G   K   D   A   A   A   A   R   Y   I   F   T   E   L   N   D   I
```

```
          1090       1100       1110       1120       1130       1140
ACAAAGGCTATTTTCAACCCATTAGACAATCCGATTTATACCTATGTTCAAGATGATGAG
 T   K   A   I   F   N   P   L   D   N   P   I   Y   T   Y   V   Q   D   D   E
```

```
          1150       1160       1170       1180       1190
CAAACTGTTGAGCCGCAGTGGTATTTGCCAGTTTTGCCAATGATTTTG
 Q   T   V   E   P   Q   W   Y   L   P   V   L   P   M   I   L
```

(vii) basidiomycetous yeast
Origin: Rhodotorula bogoriensis

Length of sequence: 288

```
           10         20         30         40         50         60
CCTCGTCAACGCTGCCGACAACAAGGTGCGTTTTCTGTTGTCCACGACGAACGGGACGCC
  L   V   N   A   A   D   N   K
```

```
           70         80         90        100        110        120
TGAGCTGACACGAGAACGCGTGCAGGTTCGCGACCCGTCCATGGCGAACCTCAAGGTCTC
                           V   R   D   P   S   M   A   N   L   K   V   S
```

```
          130        140        150        160        170        180
GATCGACGTCGAGAAGCGCGAGATCTCAGTCTTCAACGACGGAGCCGGAATTCCGATCGA
  I   D   V   E   K   R   E   I   S   V   F   N   D   G   A   G   I   P   I   E
```

```
          190        200        210        220        230        240
GATGCACAAGAAGGAGGATATGTGGATCCCGTCGCTCATCTTCGGAGTGCTCCTGACCTC
  M   H   K   K   E   D   M   W   I   P   S   L   I   F   G   V   L   L   T   S
```

```
            250         260         270         280         290
GTCCAACTACGACGACGACGAGGACAAGGTCACCGGAGGTCGTAACGG
   S   N   Y   D   D   D   E   D   K   V   T   G   G   R   N
```

EXAMPLE 3

Identification of petroleum-decomposing yeast using type II topoisomerase gene:

The type II topoisomerase gene fragment of a yeast isolated as a petroleum-decomposing microorganism from a heavy oil-contaminated environment was amplified by PCR using a set of primers corresponding to the amino acid sequences (f) and (j) or the amino acid sequences (j) and (k). The DNA sequence of the amplified fragment was determined, which was then compared with reference sequences from standard strains of various organisms. As a result, the strain was identified to be *Candida guilliermondii*. The method utilizing the commonly employed 18S rRNA gene was unable to identify this strain.

In contrast, the invention enables rapid and precise identification and detection of unidentified organisms. In particular, the invention makes it possible to distinguish such minute differences among individuals that could not been detected by the method using the 18S rRNA gene.

While the invention has been described in detail and with reference to specific embodiments thereof, it will be apparent to one skilled in the art that various changes and modifications can be made therein without departing from the spirit and scope thereof.

This application is based on Japanese patent application No. Hei.-10-270337 filed on September 24, 1998, incorporated herein by reference.

**Claims**

1.　A method of identifying a eukaryote based on a base sequence of an intron-containing region in a type II topoisomerase gene.

2.　A method of identifying a eukaryote which comprises amplifying the DNA of the eukaryote by a polymerase chain reaction or cDNA cloning using at least one polymerase chain reaction primer or cDNA cloning probe comprising an oligonucleotide having a base sequence coding for a part of or the whole of one of the following amino acid sequences (a) to (k) that functions as a substantial primer or probe, and identifying the eukaryote based on the sequence of the amplified DNA:

> (a) Asp-Asp-Glu-Lys-Lys-Val-Thr-Gly,
> (b) Lys-Lys-Lys-Asn-Lys-Gly-Gly,
> (c) Met-Ile-Met-Thr-Asp-Gln-Asp-Gln-Asp-Gly,
> (d) Glu-Glu-Phe-Ile-Thr-Pro-Ile-Val,
> (e) Pro-Glu-Phe-Glu-Glu-Trp-Lys,
> (f) Arg-Pro-Asp-(Thr or Ser)-Tyr-Ile-Gly,
> (g) Leu-(Tyr or Leu or Phe)-Lys-Ile-Phe-Asp-Glu-Ile,
> (h) Asn-Gly-(Tyr or Phe)-Gly-Ala-Lys-Leu,
> (i) Thr-Ile-Lys-Gly-Gly-Thr-His-Val,
> (j) Asp-Ala-Asn-Lys-Ala-Gly-The-Lys, and
> (k) Val-Asn-Gly-Ala-Glu-Gly-Ile.

3.　The method of identifying a eukaryote according to claim 2, wherein the DNA to be amplified comprises an intron.

4.　A method of detecting a eukaryote based on the base sequence of an intron-containing region in a type II topoisomerase gene.

5.　A method of detecting a eukaryote which comprises amplifying DNA of the eukaryote by a polymerase chain reaction or cDNA cloning using at least one polymerase chain reaction primer or cDNA cloning probe comprising an oligonucleotide having a base sequence coding for a part of or the whole of one of the following amino acid sequences (a) to (k) that functions as a substantial primer or probe, and detecting the eukaryote based on the sequence of the amplified DNA:

(a) Asp-Asp-Glu-Lys-Lys-Val-Thr-Gly,
(b) Lys-Lys-Lys-Asn-Lys-Gly-Gly,
(c) Met-Ile-Met-Thr-Asp-Gln-Asp-Gln-Asp-Gly,
(d) Glu-Glu-Phe-Ile-Thr-Pro-Ile-Val,
(e) Pro-Glu-Phe-Glu-Glu-Trp-Lys,
(f) Arg-Pro-Asp-(Thr or Ser)-Tyr-Ile-Gly,
(g) Leu-(Tyr or Leu or Phe)-Lys-Ile-Phe-Asp-Glu-Ile,
(h) Asn-Gly-(Tyr or Phe)-Gly-Ala-Lys-Leu,
(i) Thr-Ile-Lys-Gly-Gly-Thr-His-Val,
(j) Asp-Ala-Asn-Lys-Ala-Gly-The-Lys, and
(k) Val-Asn-Gly-Ala-Glu-Gly-Ile.

6. The method of detecting a eukaryote according to claim 5, wherein the DNA to be amplified comprises an intron.

7. An oligonucleotide which comprises an oligonucleotide having a base sequence coding for a part of or the whole of a polypeptide selected from the group consisting of:

(a) Asp-Asp-Glu-Lys-Lys-Val-Thr-Gly,
(b) Lys-Lys-Lys-Asn-Lys-Gly-Gly,
(c) Met-Ile-Met-Thr-Asp-Gln-Asp-Gln-Asp-Gly,
(d) Glu-Glu-Phe-Ile-Thr-Pro-Ile-Val,
(e) Pro-Glu-Phe-Glu-Glu-Trp-Lys,
(f) Arg-Pro-Asp-(Thr or Ser)-Tyr-Ile-Gly,
(g) Leu-(Tyr or Leu or Phe)-Lys-Ile-Phe-Asp-Glu-Ile,
(h) Asn-Gly-(Tyr or Phe)-Gly-Ala-Lys-Leu,
(i) Thr-Ile-Lys-Gly-Gly-Thr-His-Val,
(j) Asp-Ala-Asn-Lys-Ala-Gly-The-Lys, and
(k) Val-Asn-Gly-Ala-Glu-Gly-Ile.

8. Use of a base sequence of an intron-containing region in a type II topoisomerase gene for identification or detection of a eukaryote.

# Fig. 1

```
TOP2_ASFB7    ------------------------------------------MEAFEISDFKEHA
TOP2_ASFM2    ------------------------------------------MEAFEISDFKEHA
TOP2_CAEEL    MYDTHDLRLPWVAFFTRKYVKAGDELTWDYQYTQDQTATTQLTCHCGAENCTGRLLKKAA
TOP2_PLAFK    ----------------------------------------------MAKNKTIE
TOP2_ARATH    ---------------------------MATKLPLQNSNAANVAKAPAKSRAAAGGKTIE
TOP2_CRIGR    --------------------------MELSPLQPVNENMQMNK-KKNEDAKKRLSIE
TOP2_DROME    ------------------------------------------MENGNKALSIE
TOP2_YEAST    -----------------------------------------MSTEPVSAS
TOPB_HUMAN    --------MAKSGGCGAGAGVGGGNGALTWVTLFDQNNAAKKEESETANKNDSSKKLSVE
TOP2_CANAL    -MSESESDYFTDGSEDDFVPTSKKSTKKNASSKSKQPLGDATNSTVSSSRSSTPKPTNAS
TOP2_SCHPO    -----------------------------------MTASEQIPLVTNNGNGNSNVS
TOPA_HUMAN    -------------------------MEVSPLQPVNENMQVNKIKKNEDAKKRLSVE
TOP2_MOUSE    -------------------------MELSPLQPVNENMLMNK-KKNEDGKKRLSIE
TOP2_RAT      -------------------------MELSPLQPVNENMLLNK-KKNEDGKKRLSVE
TOP2_CRIFA    ------------------------------------------------------MTDA
TOP2_TRYBB    -------------------------------------------------------MAEA
TOP2_TRYCR    -------------------------------------------------------MAEA
```

```
TOP2_ASFB7    KKKSMWAGALNKVT-----ISGLMG-----VFTED-EDLMALPIHRDHCPALLKIFDELI
TOP2_ASFM2    KKKSMWAGALNKVT-----ISGLMG-----VFTED-EDLMALPIHRDHCPALLKIFDELI
TOP2_CAEEL    AKYEKKSPTEHVLLRPDTYIGGVAMREDQIIWLRDSENRKMIAKEVTYPPGLLKIFDEIL
TOP2_PLAFK    ERYQKKSQIEHILLRPDTYIGSVEMHT-QLLWVWNKEKNRMVQKNITYVPGLYKIFDEII
TOP2_ARATH    EMYQKKSQLEHILLRPDTYIGSIEKHT-QTLWVYE--KDEMVQRPVTYVPGLYKIFDEIL
TOP2_CRIGR    RIYQKKTQLEHILLRPDTYIGSVELVT-QQMWVYD-EDVGINYREVTFVPGLYKIFDEIL
TOP2_DROME    QMYQKKSQLEHILLRPDSYIGSVEFTK-ELMWVYDNSQNRMVQKEISFVPGLYKIFDEIL
                                  (f)                               (g)
TOP2_YEAST    DKYQKISQLEHILKRPDTYIGSVETQE-QLQWIYDEETDCMIEKNVTIVPGLFKIFDEIL
TOPB_HUMAN    RVYQKKTQLEHILLRPDTYIGSVEPLT-QFMWVYD-EDVGMNCREVTFVPGLYKIFDEIL
TOP2_CANAL    ETYQKLSQLEHILKRPDTYIGSVEKTK-TEMWCFDAETESMVFKEVTIVPGLYKIFDEIL
TOP2_SCHPO    TQYQRLTPREHVLRRPDTYIGSIEPTT-SEMWVFDSEKNKLDYKAVTYVPGLYKIFDEII
TOPA_HUMAN    RIYQKKTQLEHILLRPDTYIGSVELVT-QQMWVYD-EDVGINYREVTFVPGLYKIFDEIL
TOP2_MOUSE    RIYQKKTQLEHILLRPDTYIGSVELVT-QQMWVYD-EDVGINYREVTFVPGLYKIFDEIL
TOP2_RAT      RIYQKKTQLEHILLRPDTYIGSVELVT-QQMWVYD-EDVGINYREVTFVPGLYKIFDEIL
TOP2_CRIFA    SKYQKLTPIDHVLLRPEMYVGSIETQS-IPMFVFDPAKGKMVWESMQVNQGLLKIVDEIL
TOP2_TRYBB    HKYKKLTPIEHVLTRPEMYIGSLDTTA-TPMFIYDEQKGHMVWETVKLNHGLLKIVDEIL
TOP2_TRYCR    SKYKKLTPIDHVLIRPEMYVGSVDTSS-SSMFVFDHEKGRMVWESLKVNHGLLKIVDEIL
```

# Fig. 2

```
TOP2_ASFB7    VNATDHER-ACHSKTK-KVTYIKISFDKG--VFSCENDGPGIPIAKHEQASLIAKRDVYV
TOP2_ASFM2    VNATDHER-ACHSKTK-KVTYIKISFDKG--VFACENDGPGIPIAKHEQASLIAKRDVYV
TOP2_CAEEL    VNAADNKA-RDSSMNR-LEVWLDRETAR----ISVWNNGSGLPVEIHPTEG------IYV
TOP2_PLAFK    VNAADVKA-REKEKSENPMTCIKIEINKENKRISVYNDGEGIPVDIHKEMN------IYV
TOP2_ARATH    VNAADNKQ-RDAKMDS-VQVVIDVEQNL----ISVCNSGAGVPVEIHQEEG------IYV
TOP2_CRIGR    VNAADNKQ-RDPKMSC-IRVTIDPENNL----ISIWNNGKGIPVVEHKVEK------MYV
TOP2_DROME    VNAADNKQ-RDKSMNT-IKIDIDPERNM----VSVWNNGQGIPVTMHKEQK------MYV
TOP2_YEAST    VNAADNKV-RDPSMKR-IDVNIHAEEHT----IEVKNDGKGIPIEIHNKEN------IYI
TOPB_HUMAN    VNAADNKQ-RDKNMTC-IKVSIDPESNI----ISIWNNGKGIPVVEHKVEK------VYV
TOP2_CANAL    VNAADNKI-RDPSMKN-IRVKIDAENNI----IEVMNDGKGIPIEMHTKEN------MYI
TOP2_SCHPO    VNAADNKV-RDPNMNT-LKVTLDPEANV----ISIYNNGKGIPIEIHDKEK------IYI
TOPA_HUMAN    VNAADNKQ-RDPKMSC-IRVTMIRKQLI-----SIWNNGKGIPVVEHKVEK------MYV
TOP2_MOUSE    VNAADNKQ-RDPKMSC-IRVTIDPENNV----ISIWNNGKGIPVVEHKVEK------IYV
TOP2_RAT      VNAADNKQ-RDPKMSC-IRVTMMRNNLI-----SIWNNGKGIPVVEHKVEK------MYV
TOP2_CRIFA    LNAADNIN-NSVRGARMTYISIKISDSGE---IMVENDGAGLPIVKSKEHK------MYI
TOP2_TRYBB    LNASDNISNRSARMTY-IRVTITDTGEI-----TIENDGAGIPIVRSREHK------LYI
TOP2_TRYCR    LNASDNIANKGGRMTY-IRVHITEAGEI-----TIENDGAGIPIVRSKEHK------LYI
              :**:*               :               *.* *:*:          :*:


TOP2_ASFB7    PEVASCFFLAGTNINKAKDCIKGGTNGVGLKLAMVHSQWAILTTADGAQKYVQQINQRLD
TOP2_ASFM2    PEVASCHFLAGTNINKAKDCIKGGTNGVGLKLAMVHSQWAILTTADGAQKYVQHINQRLD
TOP2_CAEEL    PTLVFGNLFTSSNYDDSEIKTVGGRNGYGAKLCNIFSKEFIVETVD-TRIKRRFRQKWYD
TOP2_PLAFK    PHMIFGELLTSDNYDDAEDRITGGRNGFGAKLTNIFSKEFIVQCGD-SSRKKEFKMTWSD
TOP2_ARATH    PEMIFGHLLTSSNYDDNVKKTTGGRNGYGAKLTNIFSTEFIIETAD-GKRLKKYKQVFEN
                                        ────────
                                           (h)
TOP2_CRIGR    PALIFGQLLTSSNYDDDEKKVTGGRNGYGAKLCNIFSTRFTVETAS-KEYKKMFKQTWMD
TOP2_DROME    PTMIFGHLLTSSNYNDDEKKVTGGRNGYGAKLCNIFSTSFTVETAT-REYKKSFKQTWGN
                               ────
                                (a)
TOP2_YEAST    PEMIFGHLLTSSNYDDDEKKVTGGRNGYGAKLCNIFSTEFILETAD-LNVGQKYVQKWEN
TOPB_HUMAN    PALIFGQLLTSSNYDDDEKKVTGGRNGYGAKLCNIFSTKFTVETAC-KEYKHSFKQTWMN
TOP2_CANAL    PELIFGNLLTSSNYDDDQKKVTGGRNGFGAKLCNIFSTQFEVETAD-LNMGKLYKQSWTN
TOP2_SCHPO    PELIFGNLLTSSNYDDNQKKVTGGRNGYGAKLCNIFSTEFVVETAD-KERMKKYKQTWYD
TOPA_HUMAN    PALIFGQLLTSSNYDDDEKKVTGGRNGYGAKLCNIFSTKFTVETAS-REYKKMFKQTWMD
TOP2_MOUSE    PALIFGQLLTSSNYDDDEKKVTGGRNGYGAKLCNIFSTKFTVETAS-RAYKKMFKQTWMD
TOP2_RAT      PALIFGQLLTSSNYDDDEKKVTGGRNGYGAKLCNIFSTKFTVETAS-REYKKMFKQTWMD
TOP2_CRIFA    PEMVFGHLLTSSNYNNDASSTTAGRHGYGAKLTNILSTKFSVVCRT---AGREFHMSWTD
TOP2_TRYBB    PEMVFGHLLTSSNYDDDNQNAVAGRHGYGAKLTNILSLSFSVCCRT---NGREFHMSWQD
TOP2_TRYCR    PEMVFGHLLTSSNYDDTSQNAVAGRHGYGAKLTNILSHRFSVCCRT---KGKEFHMSWHD
              * :    :::. * :.      .* :* * **  : *    :                :
```

# Fig. 3

```
TOP2_ASFB7    IIEPPTITPSREMFTRIELMPVYQELGYAEPLS------ETEQADLSAWIYLRACQCAAY
TOP2_ASFM2    IIEPPTITPSREMFTRIELMPVYQELGYAQPLS------ETEQADLSAWIYLRACQCAAY
TOP2_CAEEL    NMKKCNEAEVVEILD--ETVKDYTKVEFVPDLERFQI--DKLSDDVIDLIGRRVFEVAAT
TOP2_PLAFK    NMSKFSEPHIKNYNG----K-DYVKVTFKPDLNKFGM--TEMDDDIESLLFKRVYDLAGT
TOP2_ARATH    NMGKKSEPVITKCNK----SENWTKVTFKPDLKKFNM--TELEDDVVALMSKRVFDIAGC
TOP2_CRIGR    NMGRAGDMELKPFNG----E-DYTCITFQPDLSKFKM--QSLDKDIVALMVRRAYDIAGS
TOP2_DROME    NMGKASDVQIKDFNG-----TDYTRITFSPDLAKFKM--DRLDEDIVALMSRRAYDVAAS
TOP2_YEAST    NMSICHPPKITSYKK----GPSYTKVTFKPDLTRFGM--KELDNDILGVMRRRVYDINGS
TOPB_HUMAN    NMMKTSEAKIKHFDG----E-DYTCITFQPDLSKFKM--EKLDKDIVALMTRRAYDLAGS
TOP2_CANAL    NMSNVSKPKITTLRT----KKEYTKITFRPDLSKFDM--DCLDNDLLSVLRRRVYDLCGT
TOP2_SCHPO    NMSRKSEPVITSLKK----PDEYTKITFKPDLAKFGM--DKIDDDMVSIIKRRIYDMAGT
TOPA_HUMAN    NMGRAGEMELKPFNG-----EDYTCITFQPDLSKFKM--QSLDKDIVALMVRRAYDIAGS
TOP2_MOUSE    NMGRAGDMELKPFSG-----EDYTCITFQPDLSKFKM--QSLDKDIVALMVRRAYDIAGS
TOP2_RAT      NMGRAGDMELKPFSG-----EDYTCITFQPDLSKFKM--QSLDKDIVALMVRRAYDIAGS
TOP2_CRIFA    HMRMATTPRVSNVDPK---EKNVTRVTFMPDYAHFGFPTAAISLDMKRVLHKRIMDLAAM
TOP2_TRYBB    HMRKATAPRVSNVGTK---EKNVTRVKFLPDYERFGMKEKKISNDMKRVLYKRIMDLSAM
TOP2_TRYCR    HMRRATAPRVSNVDPK---EKNLTRVKFLPDYERFGLDANKISHDMKRVLHKRIMDLAAM
              :                            : :         . *:   : * : .


TOP2_ASFB7    VGKGTTIYYNDKPCRTGSVMALAKMYTLLSAP----------------------------
TOP2_ASFM2    VGKGTTIYYNDKPCNTGSVMALAKMYTLLSAP----------------------------
TOP2_CAEEL    LPRDVDVYLNGQKCDVDGFEDYVKMFNDSSS-----------------------------
TOP2_PLAFK    CS--VRVYLNGQRLAVKDFKSYVDLYLKDNSNDNKNNKGQNDNNNNNNNNNDENANQNND
TOP2_ARATH    LGKSVKVELNGKQIPVKSFTDYVDLYLSAANK----------------------------
TOP2_CRIGR    TK-DVKVFLNGNKLPVKGFRSYVDMYLKDKLD----------------------------
TOP2_DROME    SK-GVSVFLNGNKLGVRNFKDYIDLHIKNTDD----------------------------
TOP2_YEAST    VR-DINVYLNGKSLKIRNFKNYVELYLKSLEKK----R-----------------QLDN
TOPB_HUMAN    CR-GVKVMFNGKKLPVNGFRSYVDLYVKDKLD----------------------------
TOP2_CANAL    VK-NCNIYLNDKRLNISSFKGYVEMYVKAIKER--------------------SPEP
TOP2_SCHPO    VR-ETKVYLNNERISISGFKKYVEMYLASDTK----------------------------
TOPA_HUMAN    TK-DVKVFLNGNKLPVKGFRSYVDMYLKDKLD----------------------------
TOP2_MOUSE    TK-DVKVFLNGNMLPVKGFRSYVDLYLKDKVD----------------------------
TOP2_RAT      TK-DVKVFLNGNRLPVKGFRSYVDMYLKDKVD----------------------------
TOP2_CRIFA    FS-KIEVRLNNVPFGFQTFNDYARLYSLPGAD----------------------------
TOP2_TRYBB    FP-NIQITLNGSSFGFKSFKDYATLYSAMTPK----------------------------
TOP2_TRYCR    FP-SIEISLNGVPFAFKSFADYAMLYSSPSSS----------------------------
              : *.          .      :.
```

# Fig. 4

```
TOP2_ASFB7    NSTIHTATIKADAKPYSLH----------------PLQVAAVVSPK-FKKFE-HVSII
TOP2_ASFM2    NSTIHTTAIKADAKPYSLH----------------PLQVAAVVSPK-FKKFE-HVSII
TOP2_CAEEL    LLFLHPTPRWHVGVAKRNN----------------FFGESHVVLP---KI----VSFV
TOP2_PLAFK    NLDVSLSNEPADGTPTKNNNNNNNNNDEDEIVKIHEKQHRWEIVVSKSDGSQFQ-QVSFV
TOP2_ARATH    ---SRTED-PLPRLTEKVN----------------DRWEVCVSLS-EGQFQ-QVSFV
TOP2_CRIGR    ----ETGN-ALKVVHEQVN----------------PRWEVCLTMS-EKGFQ-QISFV
TOP2_DROME    ----DSGP-PIKIVHEVAN----------------ERWEVACCPS-DRGFQ-QVSFV
TOP2_YEAST    GEDGAAKSDIPTILYERIN----------------NRWEVAFAVS-DISFQ-QISFV
TOPB_HUMAN    -----ETGVALKVIHELAN----------------ERWDVCLTLS-EKGFQ-QISFV
TOP2_CANAL    EPQDGTIKNFTTIVHEVFN----------------DRWEVAFAVS-DGSFN-QVSFV
TOP2_SCHPO    -----PDEEPPRVIYEHVN----------------DRWDVAFAVS-DGQFK-QVSFV
TOPA_HUMAN    -----ETGNSLKVIHEQVN----------------HRWEVCLTMS-EKGFQ-QISFV
TOP2_MOUSE    -----ETGNSLKVIHEQVN----------------PRWEVCLTMS-ERGFQ-QISFV
TOP2_RAT      -----ETGNALKVVHEQVN----------------PRWEVCLTMS-EKGFQ-QISFV
TOP2_CRIFA    -----GAMPPEPFVHTGPN-----------------GSIAFVPQLTQSPKRIVGVV
TOP2_TRYBB    -----GEKPPPPYVYESKS-----------------GCVAFIPSVVPGVRRMFGVV
TOP2_TRYCR    -----GEMPPAPFVYESRN-----------------GAIAFIPSLTAGTRRIFGVV
                                                                   ...:
```

```
TOP2_ASFB7    NGVNCVKG-EHVTFLKKTINEMVIKKFQQTIKDK-NRKTTLRDSCSNIFVVIVGSIPGIE
TOP2_ASFM2    NGVNCVKG-EHVTFLKKTINEMVVKKFQQTIKDK-NRKTTLRDSCSNIFVVIVGSIPGIE
TOP2_CAEEL    NNINTEKGGSHVDYVMDKIVNIIKPIVDSKLGDP-TKSVKPAVIKNNLSIFINCLIENPS
TOP2_PLAFK    NSICTTKGGSHVNYIVEQLLSSLSKKANAKNKG--GMEIKSGHIRNHLWVFVNCLIVNPT
TOP2_ARATH    NSIATIKGGTHVDYVTSQITNHIVAAVNKKNKN---ANVKAHNVKNHLWVFVNALIDNPA
                   (i)
TOP2_CRIGR    NSIATSKGGRHVDYVADQIVSKLVDVVKKKNKG--GVAVKAHQVKNHMWIFVNALIENPT
TOP2_DROME    NSIATYKGGRHVDHVVDNLIKQLLEVLKKKNKG--GINIKPFQVRNHLWVFVNCLIENPT
                                         (b)
TOP2_YEAST    NSIATTMGGTHVNYITDQIVKKISEILKKKKKK----SVKSFQIKNNMFIFINCLIENPA
TOPB_HUMAN    NSIATTKGGRHVDYVVDQVVGKLIEVVKKKNKA--GVSVKPFQVKNHIWVFINCLIENPT
TOP2_CANAL    NSIATTSGGTHVKYVSDQIINKLVETLSKKEKGKKKLMIKPQEVRDNMFLFINCLIENPA
TOP2_SCHPO    NNISTIRGGTHVNYVANKIVDAIDEVVKKENKK---APVKAFQIKNYVQVFVNCQIENPS
TOPA_HUMAN    NSIATSKGGRHVDYVADQIVTKLVDVVKKKNKG--GVAVKAHQVKNHMWIFVNALIENPT
TOP2_MOUSE    NSIATSKGGRHVDYVADQIVSKLVDVVKKKNKG--GVAVKAHQVKNHMWIFVNALIENPT
TOP2_RAT      NSIATSKGGRHVDYVADQIVSKLVDVVKKKNKG--GVAVKADQVKNHMWIFGNAVIENPT
TOP2_CRIFA    NGVVTYNGGTHCTSAMEILETGLDSLSRSLKKD--GKVIDTNRVARHFTVLVFLIQSQPK
TOP2_TRYBB    NGVVTYNGGTHCNAAQDILTGCLDGVERELKKE--NKVMDTNRVLRHFTILVFLVQVQPK
TOP2_TRYCR    NGVVTHNGGTHCNAAQEVLQSSLESVEKALKKD--NKVIDTNRVLRHFMILVFLVQVQPK
              *.:     * *     .: :                       . :.
```

24

# Fig. 5

```
TOP2_ASFB7    WTGQRKDELSIAENVFK--THYSIPSSFLTSMT-RS--IVDILLQSISKKDN-----HKQ
TOP2_ASFM2    WTGQRKDELSIAENVFK--THYSIPSSFLTNMT-RS--IVDILLQSISKKDN-----HKQ
TOP2_CAEEL    FESQTKETLTTKAKNFG--SIFECDAKKTAEWAEQSGLIEDIVEEVLNMKKKKL--PGKR
TOP2_PLAFK    FDSQTKETLTTKPVKFG--SKCILSDKTINNVL-KSPILSNILLWAQAKAQVEL--KKKM
TOP2_ARATH    FDSQTKETLTLRQSSFG--SKCELSEDFLKKVG-KSGVVENLLSWADFKQNKDL--KKSD
TOP2_CRIGR    FDSQTKENMTLQAKSFG--STCQLSEKFIKAAI-GCGIVESILNWVKFKAQIQL--NKKC
TOP2_DROME    FDSQTKENMTLQQKGFG--SKCTLSEKFINNMS-KSGIVESVLAWAKFKAQNDI--AKTG
TOP2_YEAST    FTSQTKEQLTTRVKDFG--SRCEIPLEYINKIM-KTDLATRMFEIADANEENAL--KKSD
TOPB_HUMAN    FDSQTKENMTLQPKSFG--SKCQLSEKFFKAAS-NCGIVESILNWVKFKAQTQL--NKKC
TOP2_CANAL    FTSQTKEQLTTKVSQFGGKDKFVANDNLINRIL-KTSIVDKIRAIANANEDKAL--QKAD
TOP2_SCHPO    FDSQTKETLTTKVSAFG--SQCTLSDKFLKAIK-KSSVVEEVLKFATAKADQQL--SKGD
TOPA_HUMAN    FDSQTKENMTLQPKSFG--STCQLSEKFIKAAI-GCGIVESILNWVKFKAQVQL--NKKC
TOP2_MOUSE    FDSQTKENMTLQAKSFG--STCQLSEKFIKAAI-GCGIVESILNWVKFKAQIQL--NKKC
TOP2_RAT      FDSQTKENMTLQAKSFG--STCQLSEKFIKAAI-GCGIVESILNWVKFKAQIQL--NKKC
TOP2_CRIFA    FDSQSKARLVSTVTMPR--VPRTALDQYLAAMPFLEAHMNSMDDQLAAELNKEIGTGKRL
TOP2_TRYBB    FDSQNKARLVSTPTMPR--VPRQDVMKYLLRMPFLEAHVSTITGQLAQELNKEIGTGRRM
TOP2_TRYCR    FDSQNKARLVSVPTMPR--VPRQELMDFLLRMPFLEAHVNTVTGQLADELNKEMGAGRRM
              : .* *  :                    .                  :            .
```

```
TOP2_ASFB7    V-----------DVDKYTRARNAGGK-RAQDCMLLAAEGDSALSLLRTGLTLGKSNPSGP
TOP2_ASFM2    V-----------DVDKYTRA-NAGGK-KAQDCMLLAAEGDSALSLVRAGLTLGKSNPSGP
TOP2_CAEEL    VSVSS-----VRDIVKLEDAEWAGITGTAEKCTLILTEGDSAKALALAGLEVLGRETYG-
TOP2_PLAFK    KAGSSKARERIIGIPKLEDANDAGSK-YSQECTLILTEGDSAKTSCLAGLSIVGRDKYG-
TOP2_ARATH    GAKTG-----RVLVEKLDDAAEAGGK-NSRLCTLILTEGDSAKSLALAGRSVLGNNYCG-
TOP2_CRIGR    SAVKHN---RIKGIPKLDDANDAGSR-NSTECTLILTEGDSAKTLAVSGLGVVGRDKYG-
TOP2_DROME    GRKSSK----IKGIPKLEDANEAGGK-NSIKCTLILTEGDSAKSLAVSGLGVIGRDLYG-
TOP2_YEAST    GTRKSR----ITNYPKLEDANKAGTK-EGYKCTLVLTEGDSALSLAVAGLAVVGRDYYG-
                                   (j)
TOPB_HUMAN    SSVKYS---KIKGIPKLDDANDAGGK-HSLECTLILTEGDSAKSLAVSGLGVIGRDRYG-
TOP2_CANAL    GSRKSR----IKGQVNLVDANKAGTK-DGHNCTLILTEGLSAMNLAVAGLSVVGRDYYG-
TOP2_SCHPO    GGLRSR----ITGLTKLEDANKAGTK-ESHKCVLILTEGDSAKSLAVSGLSVVGRDYYG-
TOPA_HUMAN    SAVKHN---RIKGIPKLDDANDAGGR-NSTECTLILTEGDSAKTLAVSGLGVVGRDKYG-
TOP2_MOUSE    SAVKHT---KIKGIPKLDDANDAGSR-NSTECTLILTEGDSAKTLAVSGLGVVGRDKYG-
TOP2_RAT      SAVKHN---RIKGIPKLDDANDAGSR-NSAECTLILTEGDSAKTLAVSGLGVVGRDKYG-
TOP2_CRIFA    SS-RS----LISSITKLVDATSSRSD-GKNIRTLIVTEGDSAKALALNSLSSEQKKFCG-
TOP2_TRYBB    SSKT-----LLTSITKLVDATSTRRD-PKHTRTLIVTEGDSAKALAQNSLSSDQKRYTG-
TOP2_TRYCR    SSKS-----LISSITKLVDATTTRRD-PRFVRTLIVTEGDSAKALAQNSLSSDQKRYTG-
              :   *  :           *: :** **        .         *
```

# Fig. 6

```
TOP2_ASFB7    SFDFCGMISLGGVIMNACKKVTNITTDSGETIMVRNEQLTNNKVLQGIVQVLGLDFNCHY
TOP2_ASFM2    SFDFCGMISLGGVIMNACKKVTNITTDSGETIMVRNEQLTNNKVLQGIVQVLGLDFNCHY
TOP2_CAEEL    ------VFPLKGKLLN----VSNLDDAR----ASKNEEISN--LLR----ILGLKFE--D
TOP2_PLAFK    ------VFPLKGKLLN----VRDASFKQ----LMDNKEIQN--IFR----IMGLDIT--D
TOP2_ARATH    ------VFPLRGKLLN----VREASTTQ----ITNNKEIEN---LK---KILGLKQN---
TOP2_CRIGR    ------VFPLRGKILN----VREASHKQ----IMENAEINN--IIK----IVGLQYKKNY
TOP2_DROME    ------VFPLRGKLLN----VREANFKQ----LSENAEINN--LCK----IIGLQYKKKY
TOP2_YEAST    ------CYPLRGKMLN----VREASADQ----ILKNAEIQA--IKK----IMGLQHR---
TOPB_HUMAN    ------VFPLRGKILN----VREASHKQ----IMENAEINN--IIK----IVGLQYKKSY
TOP2_CANAL    ------CFPLRGKLLN----VREASADQ----ISKNAEINS---LK---QIIGLQHKK-V
TOP2_SCHPO    ------VFPLRGKLLN----VREASHSQ----ILNNKEIQA--IKK----IMGFTHK---
TOPA_HUMAN    ------VFPLRGKILN----VREASHKQ----IMENAEINN--IIK----IVGLQYKKNY
TOP2_MOUSE    ------VFPLRGKILN----VREASHKQ----IMENAEINN--IIK----IVGLQYKKNY
TOP2_RAT      ------VFPLRGKILN----VREASHKQ----IMENAEINN--IIK----IVGLQYKKNY
TOP2_CRIFA    ------VFPLRGKLLN----VRNKNLKR----LKTCKELQD--LFL----ALGLELG---
TOP2_TRYBB    ------VFPLRGKLLN----VRNKNLKR----LRNCKELQE--LFC----ALGLELD---
TOP2_TRYCR    ------VFPLRGKLLN----VRNKNLKR----LKNCKELQE--LFC----ALGLELG---
                       .* * ::*     * :              ::          :*:


TOP2_ASFB7    KTQEERAKLRYGCIVACVDQDLDGCGKILGLLLAYFHLFWPQLIIH--GFVKRLLTPLIR
TOP2_ASFM2    KTQEERAKLRYGCIVACVDQDLDGCGKILGLLLAYFHLFWPQLIIH--GFVKRLLTPLIR
TOP2_CAEEL    SNSITRESLRYGRLLILADQDEDGS-HIKGLIVNFIHKFWPSLVHTD-GFIQSFRTPLLK
TOP2_PLAFK    KNKDDIKGLRYGSLMIMTDQDYDGS-HIKGLLINMIHKFWPSLLKHK-GFLSEFVTPIVK
TOP2_ARATH    MKYENVNSLRYGQMMIMTDQDHDGS-HIKGLLINFIHSFWPSLLQVP-SFLVEFITPIVK
TOP2_CRIGR    EDEDSLKTLRYGKIMIMTDQDQDGS-HIKGLLINFIHHNWPSLLRH--RFLEEFITPIVK
TOP2_DROME    LTEDDLKTLRYGKVMIMTDQDQDGS-HIKGLLINFIHTNWPELLRL--PFLEEFITPIVK
                                 (c)                                    (d)
TOP2_YEAST    KKYEDTKSLRYGHLMIMTDQDHDGS-HIKGLIINFLESSFPGLLDIQ-GFLLEFITPIIK
TOPB_HUMAN    DDAESLKTLRYGKIMIMTDQDQDGS-HIKGLLINFIHHNWPSLLKH--GFLEEFITPIVK
TOP2_CANAL    YTAENIKSLRYGHIMIMTDQDQDGS-HIKGLIINFLETSFPGLLDIP-GFLLEFITPIVK
TOP2_SCHPO    KTYTDVKGLRYGHLMIMTDQDHDGS-HIKGLIINYLESSYPSLLQIP-GFLIQFITPIIK
TOPA_HUMAN    EDEDSLKTLRYGKIMIMTDQDQDGS-HIKGLLINFIHHNWPSLLRH--RFLEEFITPIVK
TOP2_MOUSE    EDEDSLKTLRYGKIMIMTDQDQDGS-HIKGLLINFIHHNWPSLLRH--RFLEEFITPIVK
TOP2_RAT      EDEDSLKTLRYGKIMIMTDQDQDGS-HIKGLLINFIHHNWPSLLRH--RFLEEFITPIVK
TOP2_CRIFA    KTYKSPAELRYQRLLVMTDQDADGS-HIKGLVINAFESLWPSLLQHNPGYISLFSTPIVK
TOP2_TRYBB    KDYTDADELRYQRILIMTDQDADGS-HIKGLVINAFESLWPSLLVRNPGFISIFSTPIVK
TOP2_TRYCR    KIYKDAEELRYQRLLVMTDQDADGS-HIKGLVINAFEALWPSLLNRNPGFISIFSTPIVK
               ***   ::  .*** **. :* **::  :.  :* *:      ::   : **:::
```

# Fig. 7

```
TOP2_ASFB7   VYE--KGK--TMPVEFYYEQEFDAWAKKQTSLVN-HTVKYYKGLAAHDTHEVKSMFKHFD
TOP2_ASFM2   VYE--KGK--TVPVEFYYEQEFDAWAKKQTSLAN-HTVKYYKGLAAHDTHEVKSMFKHFD
TOP2_CAEEL   AK---KG---DKVRSFFSMNEYRKWADVEEGGK--WKIKYYKGLGTSTSNEAREYFSDLD
TOP2_PLAFK   VQ---KG---SQEYSFFTIAEYEQWKEN-TNLLG-WKIKYYKGLGTSTDREFKQYFSDIK
TOP2_ARATH   ATR--KGT--KKVLSFYSMPEYEEWKESLKGNATGWDIKYYKGLGTSTAEEGKEYFSNLG
TOP2_CRIGR   VS---KN---KQELAFYSLPEFEEWKSSTPNHKK-WKVKYYKGLGTSTSKEAKEYFADMK
TOP2_DROME   ATK--KN----EELSFYSLPEFEEWKNDTANHHT-YNIKYYKGLGTSTSKEAKEYFQDMD
                                     (e)
TOP2_YEAST   VSI-TKPT--KNTIAFYNMPDYEKWREE-ESHKFTWKQKYYKGLGTSLAQEVREYFSNLD
TOPB_HUMAN   AS---KN---KQELSFYSIPEFDEWKKHIENQK-AWKIKYYKGLGTSTAKEAKEYFADME
TOP2_CANAL   VTVKARGAGGKRVIPFYTMPEFEHWRDT-EGKQCRWTQKYYKGLGTSTPMEAREYFTALD
TOP2_SCHPO   CT---RG---NQVQAFYTLPEYEYWKEANNNGRG-WKIKYYKGLGTSDHDDMKSYFSDLD
TOPA_HUMAN   VS---KN---KQEMAFYSLPEFEEWKSSTPNHKK-WKVKYYKGLGTSTSKEAKEYFADMK
TOP2_MOUSE   VS---KN---KQEIAFYSLPEFEEWKSSTPNHKK-WKVKYYKGLGTSTSKEAKEYFADMK
TOP2_RAT     VS---KN---KQEIAFYSLPEFEEWKSTNPNHKK-WKVKYYKGLGTSTSKEAKEYFANMK
TOP2_CRIFA   IKVNGKA---KEVVAFHSFRDFHRWQRANPNAR--YSAKYYKGLGTSTTAEGKEYFADME
TOP2_TRYBB   AR--LRD---KSVVSFFSMKEFHKWQRSNANTP--YTCKYYKGLGTSTTAEGKEYFKDME
TOP2_TRYCR   VR--LRD---KSTHSFFSLKEFHKWQKTHGNVS--YTAKYYKALGTSTTAEGKEYFKDMD
                  :        *.    :: *    .     ****.*.:   : :. * :


TOP2_ASFB7   NMVYTFTLD-DSAKELFHIYFG-GESELRKRELCTGVVPLTETQT------------QSI
TOP2_ASFM2   NMVYTFTLD-DSAKELFHIYFG-GESELRKRELCTGVVPLTETQT------------QSI
TOP2_CAEEL   HHTVNFKYTGTTDDDAIRMAFDRDKSDERK-E----WIRRSENE-------------ITN
TOP2_PLAFK   NHKIMFLWTGDRDGDSIDMAFSKKRIEDRK-----LWLQNFILGS------------YVD
TOP2_ARATH   LHKKDFVWEDEQDGEAIELAFSKKKIEARK-N----WLSSYVPGN------------HLD
TOP2_CRIGR   RHRIQFKYSGPEDDAAISLAFSKKQVDDRK-E----WLTHFMEDRRQRKLLGLPEDYLYG
TOP2_DROME   RHRILFKYDGSVDDESIVMAFSKKHIESRK-----VWLTNHMDEVKRRKELGLPERYLYT
TOP2_YEAST   RHLKIFHSLQGNDKDYIDLAFSKKKADDRK-E----WLRQYEPGT------------VLD
TOPB_HUMAN   RHRILFRYAGPEDDAAITLAFSKKKIDDRK-E----WLTNFMEDRRQRRLHGLPEQFLYG
TOP2_CANAL   RHLKRFHALQGEDKDYIDLAFSKKKADERK-E----WLQGFLPGT------------HLD
TOP2_SCHPO   RHMKYFHAMQEKDAELIEMAFAKKKADVRK-----EWLRTYRPG------------IYMD
TOPA_HUMAN   RHRIQFKYSGPEDDAAISLAFSKKQIDDRK-E----WLTNFMEDRRQRKLLGLPEDYLYG
TOP2_MOUSE   RHRIQFKYPGPEDDAAISLAFSKKQVDDRK-E----WLTNFMEDRRQRKLLGLPEDYLYG
TOP2_RAT     RHRIQFKYSGPEDDAAISLAFSKKQVDDRK-E----WLTNFMEDRRQRKLLGLPEDYLYG
TOP2_CRIFA   RNVMRLVVE-PKDHRLLDSVFDSAEVEWRK-E----WMSKANAFQG---------EVDID
TOP2_TRYBB   KHTMRLLVD-RSDHKLLDNVFDSQEVEWRK-----DWMTKANAFTG---------EVDID
TOP2_TRYCR   KHTMRLVVE-RNDHKLLDSVFDSQEVEWRK-----DWMTKANAYTG---------EVDID
                  :          :   * . : **        :
```

## Fig. 8

```
TOP2_ASFB7    HSVRRIPCSLHLQVDTKAYKLDAIERQIPNFLDGMTRARRKILAGGVKCFASNNR-ERKV
TOP2_ASFM2    HSDRQIPCSLHLQVDTKAYKLDAIERQIPNFLDGMTRARRKILAGGLKCFASNNR-ERKV
TOP2_CAEEL    EDDEQKKARMSDGVEDLQRFESQRIQSIPSLIDGLKPSQRKILWTLLNNMDEST--EIKV
TOP2_PLAFK    HKEKDLSYYDFVNKELIYYSRYDTERSIPNIMDGWKPGQRKVLYG---CFKRNLRNECKV
TOP2_ARATH    QRQPKVTYSDFVNKELILFSMADLQRSIPSMVDGLKPGQRKILFV---AFKKIARKEMKV
TOP2_CRIGR    QTTTYLTYNDFINKELILFSNSDNERSIPSMVDGLKPGQRKVLFT---CFKRNDKREVKV
TOP2_DROME    KGTKSITYADFINLELVLFSNADNERSIPSLVDGLKPGQRKVMFT---CFKRNDKREVKV
TOP2_YEAST    PTLKEIPISDFINKELILFSLADNIRSIPNVLDGFKPGQRKVLYG---CFKKNLKSELKV
TOPB_HUMAN    TATKHLTYNDFINKELILFSNSDNERSIPSLVDGFKPGQRKVLFT---CFKRNDKREVKV
TOP2_CANAL    PEITEIPISDFINKEFILFSMSDNVRSIPSVLDGFKPGQRKVLYG---CFKKKLRSEIKV
TOP2_SCHPO    YTQPQIPIDDFINRELIQFSMADNIRSIPSVVDGLKPGQRKVVYY---CFKRNLVHETKV
TOPA_HUMAN    QTTTYLTYNDFINKELILFSNSDNERSIPSMVDGLKPGQRKVLFT---CFKRNDKREVKV
TOP2_MOUSE    QSTSYLTYNDFINKELILFSNSDNERSIPSMVDGLKPGQRKVLFT---CFKRNDKREVKV
TOP2_RAT      QTTMYLTYNDFINKELILFSNSDNERSIPSMVDGLKPGQRKVLFT---CFKRNDKREVKV
TOP2_CRIFA    RSKKLLTIGDFVHKEMVHFALVGNARAIPHCVDGLKPSQRKILWA---MLKRHSSEAAKV
TOP2_TRYBB    RSKKMLTVTDFVHKEMVHFALVGNARALAHSVDGLKPSQRKIIWA---LMRRSGNEAAKV
TOP2_TRYCR    RSKKTLTVPDFVHKEMVHFALAGNARALAHAVDGLKPSQRKILWA---IMRRSGNESAKV

                     :            : :.  :** . .:**::      :        **


TOP2_ASFB7    FQFGGYVADHMFYHH-GDMSLNTSIIKAAQYYPGSSHLYPVFIGIGSFGSRHLGGKDAGS
TOP2_ASFM2    FQFGGYVADHMFYHH-GDMSLNTSIIKAAQYYPGSSHLYPVFIGIGSFGSRHLGGKDAGS
TOP2_CAEEL    SQLAGAVAHRQSYHH-GEESLVRTIIRMGQTFCGSS-NLPLLQPIGQFGTRHEGGNDAAS
TOP2_PLAFK    AQLVGYIAEHSAYHHHGESSLQQTIINMAQTFVGSN-NINFLEPCGQFGSRKEGGKDASA
TOP2_ARATH    AQLVGYVSLLSAYHH-GEQSLASAIIGMAQDYVGSN-NINLLLPNGQFGTRTSGGKDSAS
TOP2_CRIGR    AQLAGSVAEMSSYHH-GEMSLMMTIINLAQNFVGSN-NLNLLQPIGQFGTRLHGGKDSAS
TOP2_DROME    AQLSGSVAEMSAYHH-GEVSLQMTIVNLAQNFVGAN-NINLLEPRGQFGTRLSGGKDCAS
TOP2_YEAST    AQLAPYVSECTAYHH-GEQSLAQTIIGLAQNFVGSN-NIYLLLPNGAFGTRATGGKDAAA
TOPB_HUMAN    AQLAGSVAEMSAYHH-GEQALMMTIVNLAQNFVGSN-NINLLQPIGQFGTRLHGGKDAAS
TOP2_CANAL    AQLAGYVSENTGYHH-GEQSLVQTIIGLAQNFVGSN-NINVLKPNGSFGSRAAGGKDFSA
TOP2_SCHPO    SRLAGYVASETAYHH-GEVSMEQTIVNLAQNFVGSN-NINLLMPNGQFGTRSEGGKNASA
TOPA_HUMAN    AQLAGSVAEMSSYHH-GEMSLMMTIINLAQNFVGSN-NLNLLQPIGQFGTRLHGGKDSAS
TOP2_MOUSE    AQLAGSVAEMSSYHH-GEMSLMMTIINLAQNFVGSN-NLNLLQPIGQFGTRLHGGKDSAS
TOP2_RAT      AQLAGSVAEMSSYHH-GEMSLMMTIINLAQNFVGSN-NLNLLQPIGQFGTRLHGGKDSAS
TOP2_CRIFA    AQLSGYISEVSSFHH-GEASLQETIVKMAQNFTGGN-NINLLVPEGQFGSRQQLGNDHAA
TOP2_TRYBB    AQLSGYISEASAFHH-GETSLQETMIKMAQSFTGGN-NVNLLVPEGQFGSRQQLGNDHAA
TOP2_TRYCR    AQLSGYISEVSAFHH-GEMSLQETIIKMAQNFTGGN-NINLLIPEGQFGSRQQLGNDHAA

               ::    ::   :** *: :: ::: .* : *..   .: * **:*  *:: .:
```

# Fig. 9

```
TOP2_ASFB7    PRYISVQLASEFIKTMFPAEDSWLLPYVFEDGQRAEPEYYVPVLPLAIMEYGANPSEGWK
TOP2_ASFM2    PRYISVQLASEFIKTMFPAEDSWLLPYVFEDGQRAEPEYYVPVLPLAIMEYGANPSEGWK
TOP2_CAEEL    ARYIFTALA-PTTRLLFPQADDDLLQKNVEEGMVVEP-TLCPIVPLILINGTEGIGTGWS
TOP2_PLAFK    ARYIFTKLA-SSTRSIFNEYDDPILKYLNEEGQKIEPQYYIPVIPTILVNGCEGIGTGYS
TOP2_ARATH    ARYIFTKLS-PVTRILFPKDDDLLLDYLNEDGQRIEPTWYMPIIPTVLVNGAEGIGTGWS
TOP2_CRIGR    PRYIFTMLS-PLTRLLFPPKDDHTLKFLYDDNQRVEPEWYIPIIPMVLINGAEGIGTGWS
TOP2_DROME    ARYIFTIMS-PLTRLIYHPLDDPLLDYQVDDGQKIEPLWYLPIIPMVLVNGAEGIGTGWS
TOP2_YEAST    ARYIYTELN-KLTRKIFHPADDPLYKYIQEDEKTVEPEWYLPILPMILVNGAEGIGTGWS
                                                             (k)

TOPB_HUMAN    PRYIFTMLS-TLARLLFPAVDDNLLKFLYDDNQRVEPEWYIPIIPMVLINGAEGIGTGWA
TOP2_CANAL    ARYIFTELS-EITRKIFNPLDDPLYTYVQDDEQTVEPEWYLPVLPMILVNGAEGIGTGWS
TOP2_SCHPO    SRYLNTALS-PLARVLFNSNDDQLLNYQNDEGQWIEPEYYVPILPMVLVNGAEGIGTGWS
TOPA_HUMAN    PRYIFTMLS-SLARLLFPPKDDHTLKFLYDDNQRVEPEWYIPIIPMVLINGAEGIGTGWS
TOP2_MOUSE    PRYIFTMLS-PLARLLFPPKDDHTLRFLYDDNQRVEPEWYNPINTMVLINGAEGIGTGWS
TOP2_RAT      PRYIFTMLS-PLARLLFPSKDDHTLRFLYDDNQRVEPEWYIPIIPMVLINGAEGIGTGWS
TOP2_CRIFA    PRYIFTKLS-RFARLLFPEDDDPLLDYIDEEGTMVEPNHYVPILPLLLCNGAVGIGFGFA
TOP2_TRYBB    PRYIFTKLS-KVARLLFPSEDDPLLDYIVEEGQQVEPNHYVPILPLLLCNGSVGIGFGFS
TOP2_TRYCR    ARYIFTKLS-SLARILFPSEDEPLLDYVTEEGQQVEPNHYVPILPLLLCNGSVGIGFGFA
              .**: . :     : :: *.        ::    **   *: . : :   . . *:
```

# Fig. 10

```
CG-1 ATOP2    1:GACGGCTCTCACATCAAGGGCCTTCTCATCAACTTCATGCACCACTTCTGGCCCAACCTC 60
IS-2 ATP2     1:........................................................... 60
IS-4 ATP2     1:........................................................... 60
IS-6 ATP2     1:........................................................... 60
IS-11 ATP2    1:........................................................... 60
IS-12 ATP2    1:......................................................T...... 60
IS-14 ATP2    1:......................................................T...... 60
IS-8 ATP2     1:...............................................T.........T... 60
IS-10 ATP2    1:...............................................T.........T... 60
IS-15 ATP2    1:...............................................T..............T 60
                ************************************** ********** ** **


CG-1 ATOP2   61:CTGAAGTACAATGTCATTGAGGAGTTCATCACACCCATTGTCAAGGTATAGGATACATGT 120
IS-2 ATP2    61:........................................................... 120
IS-4 ATP2    61:........................................................... 120
IS-6 ATP2    61:........................................................... 120
IS-11 ATP2   61:........................................................... 120
IS-12 ATP2   61:........................................................... 120
IS-14 ATP2   61:........................................................... 120
IS-8 ATP2    61:..C.....................................G.................. 120
IS-10 ATP2   61:..C.....................................G.................. 120
IS-15 ATP2   61:..C...................................CT.................. 120
                ** **************************************** ******* ********


CG-1 ATOP2  121:ACTGTCATTGTAATGTGTATGT-AA----TAGAAAG------ATGAAAGTGAATGCTGTC 169
IS-2 ATP2   121:......................-..----.......------................. 169
IS-4 ATP2   121:......................-..----.......------................. 169
IS-6 ATP2   121:......................-..----.......------................. 169
IS-11 ATP2  121:......................-..----.......------................. 169
IS-12 ATP2  121:......................-..----.......------................. 169
IS-14 ATP2  121:......................-..----.......------................. 169
IS-8 ATP2   121:.---C...CA....A.......T..GTTT......ATATAC........A.G..T...A 177
IS-10 ATP2  121:.---C...CA....A.......T..GTTT......ATATAC........A.G..T...A 177
IS-15 ATP2  121:.GCA.......G..A.......A..GTTT.......------..............T.... 174
                *   *** * ** ******* **    *******    ******** * ** ***


CG-1 ATOP2  170:CTTTGA-GGTTGA--AAAATACTTG--G---A------AGTATAAAATGTATTGTAGGTC 215
IS-2 ATP2   170:......-.....A.--....A....---.---.------................... 214
IS-4 ATP2   170:......-.......--...........--.---.------................... 215
IS-6 ATP2   170:......-.......--...........--.---.------.........A.......... 215
IS-11 ATP2  170:......-.......--...........--.---.------................... 215
IS-12 ATP2  170:......-.......--...........--.---.------................... 215
IS-14 ATP2  170:......-.......--...........--.---.------................... 215
IS-8 ATP2   178:..A...G......GGTTG.A.GA..CTTGAA.GTATAA.A..-C.TG.A.G...C..... 236
IS-10 ATP2  178:..A...G......GGTTG.A.GA..CTTGAA.GTATAA.A..-C.TG.A.G...C..... 236
IS-15 ATP2  175:.......-.......--...G.G....CA.ACA.GT-TGC.........A.C...C..... 230
                ** *** **** *       *       *       * **   * * * *** *****


CG-1 ATOP2  216:ACAAAAGGAAAAACAGAGCTATCTTTCTACAGCTTACC           253
IS-2 ATP2   215:.......................................            252
IS-4 ATP2   216:.......................................            253
IS-6 ATP2   216:.......................................            253
IS-11 ATP2  216:.......................................            253
IS-12 ATP2  216:................G......................            253
IS-14 ATP2  216:.......................................            253
IS-8 ATP2   237:.......................................            274
IS-10 ATP2  237:.......................................            274
IS-15 ATP2  231:..T....................................            268
                ** ******************** ******************
```